# EUROPEAN PATENT APPLICATION

(11) **EP 3 395 321 A2**
(43) Date of publication of application: **31.10.2018**
(21) Application number: 18171401.5
(22) Date of filing: 01.04.2014
(51) Int. Cl.: A61K 8/14, A61K 8/97, A61Q 5/12, A61Q 19/00, A61K 36/48, A61K 36/899, A61K 36/8998, A61K 36/185, A61K 36/21, A61K 36/488, A61Q 1/02, A61K 8/55, A61Q 5/06, A61K 9/127, A61K 8/02, A61K 8/9789

(54) **COMPOSITIONS FOR PERSONAL CARE COMPRISING BOTANICAL POWDER AS ACTIVE COMPOUND AND DELIVERY SYSTEM**

(30) Priority: 02.04.2013 US 201361807554 P; 09.10.2013 US 201361888649 P
(62) Divisional of application: 14723234.2
(71) Applicant: Evonik Degussa GmbH, 45128 Essen (DE)
(72) Inventor: Beyer, Monika, 61130 NIDDERAU-OSTHEIM (DE); Goddard, Richard Joseph, Fogelsville, PA Pennsylvania 18051 (US); Getzat, Caroline, 36381 Schluchtern (DE); Munk, Christian, 36396 STEINAU-MARBORN (DE); Noor, Mussarat, Pittstown, NJ New Jersey 08867 (US); Sacher, Michael, 36381 SCHLUCHTERN-BREITENBACH (DE)

(57) **Abstract**

The disclosure relates to compositions comprising at least one of Active Compounds and Botanical Powders and a Delivery System, wherein at least a portion of the Botanical Powders are encapsulated in the Delivery System. Furthermore the present disclosure relates to a method for preparing compositions with reduced sedimentation of the Botanical Powders, and a Delivery System, wherein at least a portion of the Botanical Powders are encapsulated in the Delivery System. Furthermore, the present disclosure relates to compositions with reduced sedimentation comprising Botanical Powders, and a delivery system, wherein at least a portion of the Botanical Powders are encapsulated in the delivery system, for use in Cosmetics.

## Description

This application claims the benefit of U.S. Patent Application No. 61/807554, filed on 02 April 2013 and Application No. 61/888649, filed on 09 October 2013. The disclosure of Application Nos. 61/807554 and 61/888649 are hereby incorporated by reference.

### BACKGROUND OF THE INVENTION

The present disclosure relates generally to the field of cosmetic actives and delivery systems and more particularly to compositions comprising botanical powders and a delivery system and their use in personal care compositions.

It is known that a variety of botanical substances are used in the preparation of cosmetic and other personal care products. These botanical substances can provide a range of functionality (e.g.: cosmetic actives, emollients, and emulsifiers) and benefits as cosmetic ingredients.

To obtain the desired functionality and benefits from the various substances derived from the botanical powders, a variety of processes may be used to extract and purify the botanical substances. While such processes may be useful to increase the purity or modify the functionality of the botanical powders, they can also add cost to the cosmetic ingredient and affect the properties of the resultant powder. Use of botanical powders that are less processed may be desirable in some circumstances, but such powders can be more difficult to incorporate into personal care compositions, particulary if the botanical powders contain an insoluble fraction that can produce an undesirable sediment.

U.S. Patent Pub No 2006/0257386 and Korean Patent Application Nos KR2009040517A and KR20070080062A disclose certain botanical extracts (the previously identified patent publications are hereby incorporated by reference). There is a need in this art for compositions and methods for providing botantical powders wherein the botanical powders can be incorporated into a personal care composition in a manner that avoids botanical powder sedimentation.

### BRIEF SUMMARY OF THE INVENTION

The instant invention can solve problems associated with sedimentation of Botanical Powders (defined below) by incorporating the Botanical Powders into a suitable Delivery System (defined below). Exemplary embodiments of the present invention have been found to overcome this problem and offer other benefits by providing compositions of Botanical Powders and a Delivery System (defined below Botanical Powder-Based Delivery System) with reduced sedimentation. It is also advantageous to incorporate botanical powders within Delivery Systems (e.g.,Liposomes and Cerasomes as defined below) in order to improve the availability and effectiveness of the Botanical Powders as Personal Care and Cosmetic compositions. These compositions provide desirable characteristics for use in Cosmetic and Personal Care compositions such as moisturization of keratinous substrates. Other potential benefits of Botanical Powder-Based Delivery Systems include, without limitation, improved skin appearance, such as radiance, brightness, and smoothness, promotion of a more youthful and healthy skin appearance, a more even skin tone, promotion of clear skin, reduction in the appearance of wrinkles, fine lines, and stretch marks, supports the skin's natural collagen and elastin synthesis, supports the skin in its defense against environmental aggressors, reduced skin redness and irritation, soothes the skin, reduction of Trans-Epidermal Water Loss (TEWL) on skin, improved hair strength, conditioning and appearance.The present invention relates to compositions with reduced sedimentation comprising Botanical Powders and a Delivery System, wherein at least a portion of the Botanical Powders are encapsulated in the Delivery System. Furthermore the present invention relates to a method for preparing compositions with reduced sedimentation of the Botanical Powders, and a Delivery System, wherein at least a portion of the Botanical Powders are encapsulated in the Delivery System. Furthermore, the present invention relates to compositions with reduced sedimentation comprising Botanical Powders, and a delivery system, wherein at least a portion of the Botanical Powders are encapsulated in the delivery system, for use in Cosmetics.

The instant invention can also improve topical epidermal delivery of Actives (defined below) by incorporating the Actives into a suitable Delivery System (defined below). Exemplary embodiments of the present invention have been found to achieve such improvements and offer other benefits by providing compositions of active compounds and a Delivery System. It is also advantageous to incorporate actives (and if, desired, along with at least one Botanical Powder), within Delivery Systems (e.g.,Liposomes and Cerasomes as defined below) in order to improve the availability and effectiveness of the actives for use in Personal Care and Cosmetic compositions.

The following definitions are provided and used throughout this disclosure: "Botanical Powder" means and comprises particulate material obtained from any plant, and includes, without limitation, material from the entire plant, root, stem, seeds, flowers, leaves and any other constituents of the plant, and includes material derived from chemical or physical processes including, without limitation, fermenting, grinding, hydrolyzing, pulverizing, milling, extraction and drying, among other processes.

"Delivery System" means and comprises at least one of Cerasome, Liposome I, and Liposome II including, without limitation, phospholipid, ceramide, glycosphingolipid, solvent and other ingredients of the system.

"Botanical Powder-Based Delivery System" means and comprises a Botanical Powder combined with a Delivery System and includes, without limitation, compositions of the Botanical Powder and the Delivery System with reduced sedimentation (defined below).

"Personal Care" means and comprises any toiletry and topical care products including, without limitation, leave-on personal care products (i.e., products that are left on keratinous substrates after application); rinse-off personal care products (i.e., products that are washed or rinsed from keratinous substrates during or within a few minutes of application); at least one of shampoos; chemical and non-chemical hair curling and hair straightening products; hair style maintaining and hair conditioning products; lotions and creams for nails, hands, feet, face, scalp and/or body; hair dye; face and body makeup; nail care products; astringents; deodorants; antiperspirants; anti-acne; antiaging; depilatories; colognes and perfumes; skin protective creams and lotions (such as sunscreens); skin and body cleansers; skin conditioners; skin toners; skin firming compositions; skin tanning and lightening compositions; liquid soaps; bar soaps; bath products; shaving products; and oral hygiene products (such as toothpastes, oral suspensions, and mouth care products). The form of such Personal Care products is not limited and may be, without limitation, a liquid, gel, spray, emulsion (such as lotions and creams), shampoo, pomade, foam, tablet, stick (such as lip care products), makeup, suppositories, among others, any of which can be applied to the skin or hair and which typically are designed to remain in contact there with until removed, such as by rinsing with water or washing with shampoo or soap. Other forms could be gels that can be soft, stiff, or squeezable. Emulsions can be oil-in-water, water-in-oil, water-and-silicone, or multiphase. Sprays can be non-pressurized aerosols delivered from manually pumped finger-actuated sprayers or can be pressurized aerosols such as mousse, spray, or foam forming formulation, where a chemical or gaseous propellant is used. Personal Care products are topically applied only to keratinous substrates, particularly skin and hair, of humans or other animals.

"Cosmetic" means and comprises a Personal Care product comprising a powder, lotion, lipstick, rouge, or other preparation for affecting the appearance of or beautifying the face, skin, hair, nails or other keratinous materials. Cosmetic products are topically applied only to keratinous substrates, particularly skin and hair, of humans and animals.

"Reduced sedimentation" or "sedimentation reduction" or "reduction in sedimentation" means gravimetrically determined measurement wherein a reduction in sedimentation of a composition is greater than about 20%, in other cases greater than about 35%, and in yet other cases greater than about 50%.

"Active", "Actives", "Active Compound", "Cosmetic Active", "Cosmetic Active Ingredient" or "Active Ingredient" (including uncapitalized variants of the foregoing) means and comprises compounds, substances, molecules, and mixtures that are used in cosmetic and personal care products in order to interact with or protect the skin or hair in a manner that brings about a favorable and intended change in one or more cosmetic skin or hair parameters (e.g. wrinkles, elasticity, for example). It may be desirable in some cases that the Active Compound is transported into the epidermal layer in order to interact with or protect the epidermis and deeper skin layers.

In one embodiment of the invention, the invention comprises a composition comprising at least one Botanical Powder and at least one Delivery System.

In one embodiment of the invention, the invention comprises at least one active compound and at least one Delivery System. In some cases, this embodiment further comprises at least one Botanical Powder.

In another embodiment of the invention, the Botanical Powder comprises at least one member selected from the group consisting of alfalfa (genus Medicago), amaranth (genus Amaranthus), argan (genus Argania), arrowroot, barley (genus Hordeum), lupine (genus Lupinus), oat (genus Avena), rice (genus Oryza), soya (genus Glycine), pea (genus Pisum), powders of cinnamon extract, powders of Citrus medica limonum extract, powders of Cucumis, powders of Glycyrrhiza, and wheat (genus Triticum) and mixtures thereof.

In one embodiment of the invention, the invention comprises a composition wherein the Botanical Powder comprises materials derived from any one or more parts of, or whole plant of, any plant within the group.

In another embodiment of the invention, the foregoing material comprises at least one of hydrolyzed extracts, partially hydrolyzed extracts, a substance derived from the Botanical Powder and combinations thereof.

In a further embodiment of the invention, the Delivery System comprises at least one member selected from the group consisting of Cerasome, Liposome I and Liposome II.

In one embodiment of the invention, the Cerasome comprises at least one of Ceramides and Glycosphingolipids.

In another embodiment of the invention, the foregoing compositions further comprising at least one solvent selected from the group consisting of ethanol, 1,2-propanediol, 1,3-propanediol, 1,3-butylene glycol, and glycerol.

In a further embodiment of the invention, the composition is essentially free of Liposome when the Botanical Powder comprises at least one of Terminaliae fructus extract, Glycyrrhiza, Rosmarinus officinalis, Centella, Echinacea and Alpinia.

In one embodiment of the invention, the invention relates to a Personal Care product comprising the Botanical Powder Based Delivery System.

In another embodiment of the invention, the invention relates to a Cosmetic product comprising the Botanical Powder Based Delivery System.

In any of the foregoing embodiments, the invention relates to a composition that has reduced sedimentation.

A further embodiment of the invention relates to using a Delivery System to reduce sedimentation in a Botanical Powder-Based Delivery System.

The various embodiments of the invention can be used alone or in combination.

### DETAILED DESCRIPTION OF THE INVENTION

Certain embodiments of the invention are directed to compositions with reduced sedimentation comprising a Botanical Powder and a Delivery System, wherein at least a portion of, for example, a Botanical Powder is encapsulated in the Delivery System. The Delivery System of the present invention comprises vesicular compositions including membrane-forming lipids such as double-layer membrane vesicles. Liposomes are an example of the double-layer and also multi-layer vesicles. The Botanical Powder can be present both in the interior of the vesicles in a solution or dispersion, and also incorporated into or between the layers. The membrane-forming lipids of the Delivery System comprise the membrane-forming lipids from the groups of naturally-derived or synthetic phospholipids, naturally-derived or synthetic ceramides and naturally-derived or synthetic glycosphingolipids, and mixtures thereof. The Botanical Powder is combined with the Delivery System in order to obtain a Botanical Powder-Based Delivery System which in turn can be combined with other ingredients to produce a Personal Care product.

### Botanical Powders

The term "Botanical Powders" as used herein means and comprises, without limitation, a substance or composition containing or obtained from a plant material in the form of fine, loose, solid particles of the foregoing. Botanical Powders may be obtained by a variety of processes known to those skilled in the art, that may include one or more of, harvesting, cleaning, extraction, drying, crushing, grinding, pulverizing, and milling. In certain embodiments the Botanical Powder comprises a powder comprising fine, loose, solid particles with a mean particle diameter of from about 0.25 to about 500 microns, in some cases from about 2 to about 350 microns, and in other cases from about 10 to about 200 microns as determined by any suitable method, such as a laser diffraction particle size analyzer, including but not limited to a Microtrac S3500 using methods described in its operating manual.

In some cases a Botanical Powder comprises a material that is obtained through one or more processes that include extraction, maceration or percolation of the plant material with a suitable solvent and, subsequently, by predominate or complete removal of the solvent (e.g. by air drying, spray drying, vacuum oven drying, fluid-bed drying or freeze-drying, and optional washing of this dry extract in at least one suitable solvent), and botanical powders obtained through such cases are sometimes also referred to as botanical powder extracts or dry botanical extracts. Solvents suitable for extraction, percolation or maceration are known to those experienced in the art. Acetone, chloroform, ethyl acetate, lower alkanols with 1 to 4 carbon atoms, alcohols or a mixture of these and water are particularly suited. Carbon dioxide in fluid or super-critical form and pressurized gases with solvent properties are also suitable as extraction agents. The amount of solvent used for extraction will typically comprise about 1 to about 100 times the amount of the starting botanical material, and in some cases about 1 to about 10 times the amount of the starting botanical material and, if desired, an amount of the solvent of about 15 wt% or less, in some cases of about 1 wt% or less, and in other cases of about 0.1 wt% or less, can remain in the Botanical Powder. In some embodiments the botanical powder extracts are obtained from the seeds of the plants, and in other cases the botanical powder extracts can be obtained from other parts of the plant including one or more of the leaves, stems, and roots, or of the entire plant.

In other embodiments Botanical Powders include those obtained by grinding the seeds or grains of plants, and in other cases other parts of plants including one or more of the leaves, stems, and roots, or of the entire plant and optional washing of this dry powders in at least one suitable solvent.

In yet other embodiments the Botanical Powder includes for example flours, colloidal flours, and starch and malt powders and dry extracts.

In another embodiment of the invention, the Botanical Composition comprises "Botanical Powders" that comprise powders and dry extracts from any cultivated plants, any wild plants, any plant species, any plant intra-and interspecies, any plant varieties, any plant genotypes and all plant cultivars of plants including, but not limited to, soya (genus Glycine), pea (genus Pisum), lupin (genus Lupinus), alfalfa (genus Medicago), amaranth (genus Amaranthus), oat (genus Avena), licorice (genus Glycyrrhiza), chick pea (genus Cicer), lucerne (genus Medicago), horse bean (genus Vicia), lentil (genus Lens), bean (genus Phaseolus), sunflower (genus Helianthus), sesame (genus Sesamum), rapeseed (genus Brassica), argan (genus Argania), sorghum (genus Sorghum), fabaceae (genus Faba), acacia (genus Acacia), pagoda (genus Robinia and Sophora), carub (genus Pseudoacacia), wheat (genus Triticum), maize (genus Zea), barley (genus Hordeum), rye (genus Secale), rice (genus Oryza), millet, buckwheat (genus Fagopyrum), potato (genus Solanum), clover (genus Trifolium), peanut (genus Arachis), mesquite (genus Prosopis), psyllium (genus Plantago), arrowroot flour, kiwifruit (genus Actinidia), baobab (genus Adansonia), sandalwood (genus Adenanthera), horse chestnut (genus Aesculus), guinea pepper (genus Aframomum), chocolate vine (genus Akebia), candlenut (genus Aleurites), onion (genus Allium), cardamom (genus Amomum), dill (genus Anethum), angelica (genus Angelica), annona (genus Annona), celery (genus Apium), araucaria (genus Araucaria), greater burdock (genus Arctium), betel palm (genus Areca), mugwort (genus Artemisia), chamomile or dog-fennel (genus Anthemis), St. John's-wort (genus Hypericum), breadfruit (genus Artocarpus), milkvetch (genus Astragalus), neem (genus Azadirachta), desert date (genus Balanites), winter melon (genus Benincasa), Brazil nut (genus Bertholletia), lipstick plant (genus Bixa), borage (genus Borago), balltree or laurelwood (genus Calophyllum), tea (genus Camellia), hemp (genus Cannabis), balloon plant (genus Cardiospermum), safflower (genus Carthamus), caraway (genus Carum), hickory (genus Carya), cinnamon tree (genus Cassia), sweet chestnut (genus Castanea), oriental bittersweet (genus Celastrus), St. John's-bread (genus Ceratonia), quince (genus Chaenomeles), goosefoots and quinoa (genus Chenopodium), colocynth (genus Citrullus), citrus plants (genus Citrus), coffee beans (genus Coffea), coixseeds (genus Coix), cola nut (genus Cola), coriander (genus Coriandrum), common hazel (genus Corylus), swamplily (genus Crinum), cucumber (genus Cucumis), gourd (genus Cucurbita), cumin (genus Cuminum), cypress (genus Cupressus), dodder (genus Cuscuta), carrot or wild carrot (genus Daucus), tonka beans (genus Dipteryx), spikerushes (genus Eleocharis), cardamom (genus Elettaria), snuff box sea bean (genus Entada), loquat or Japanese plum (genus Eriobotrya), rucola (genus Eruca), myrtle plant (genus Eugenia), caper spurge (genus Euphorbia), common beech (genus Fagus), fennel (genus Foeniculum), strawberry (genus Fragaria), manna ash (genus Fraxinus), common snowdrop (genus Galanthus), gardenia (genus Gardenia), ginkgo (genus Ginkgo), honey locust (genus Gleditsia), melinjo (genus Gnetum), levant cotton (genus Gossypium), hibiscus plants (genus Hibiscus), woolflowers (genus Celosia), fenugreek (genus Trigonella), Brazilian cherry (genus Hymenaea), inga bean (genus Inga), ivy-leaved morning glory (genus Ipomoea), woad (genus Isatis), Persian walnut (genus Juglans), sweet pea (genus Lathyrus), cress or peppercress (genus Lepidium), oxeye daisy (genus Leucanthemum), glossy privet (genus Ligustrum), flax or linseed (genus Linum), lychee (genus Litchi), Bird's-foot Trefoil (genus Lotus), macadamia (genus Macadamia), acerola (genus Malpighia), mango (genus Mangifera), moriche palm (genus Mauritia), Indian rose chestnut (genus Mesua), four o'clock flower (genus Mirabilis), noni (genus Morinda), moringa or horseradish plant (genus Moringa), velvet bean (genus Mucuna), camu camu plant (genus Myrciaria), Indian lotus (genus Nelumbium or genus Nelumbo), longan (genus Nephelium), black cumin (genus Nigella), night-flowering jasmine (genus Nyctanthes), common evening primerose (genus Oenothera), monkey grass (genus Ophiopogon), achyranthes plant (genus Achyranthes), yarn (genus Dioscorea), Chinese boxthorn (genus Lycium), Indian trumpet flower (genus Oroxylum), Mexican yarn or yarn bean (genus Pachyrrhizus), ginseng (genus Panax), common millet (genus Panicum), passion fruit (genus Passiflora), guarana (genus Paullinia), perilla plant (genus Perilla), parsley (genus Petroselinum), phlox plants (genus Phlox), date (genus Phoenix), anise (genus Pimpinella), pine (genus Pinus), black pepper (genus Piper), pistachio (genus Pistacia), plantain (genus Plantago), inca peanut (genus Plukenetia), knotweed (genus Polygonum), almond, plum, chinese plum or bird cherry (genus Prunus), winged bean (genus Psophocarpus), babchi plant (genus Psoralea), pomegranate (genus Punica), pear or apple (genus Pyrus), ring-cupped oak (genus Quercus), radish (genus Raphanus), blackcurrant (genus Ribes), dog rose (genus Rosa), mulberry (genus Rubus), black-eyed Susan (genus Rudbeckia), chia (genus Salvia), Indian sandalwood (genus Santalum), American or Brazilian pepper (genus Schinus), five flavor berry (genus Schizandra), light red meranti (genus Shorea), milk thistle (genus Silybum), jojoba (genus Simmondsia), tamarind (genus Tamarindus), wild indigo (genus Tephrosia), cacao bean (genus Theobroma), thuja (genus Thuja), Japanese hedge parsley (genus Torilis), Indian cress or monks cress (genusTropaeolum), moth bean or mung bean (genus Vigna), grape vine (genus Vitis), bambara groundnut (genus Voandzeia), Szechuan pepper (genus Zanthoxylum), red date or Chines date (genus Zizyphus), adzuki bean (genus Vigna), amla (genus Emblica), pumpkin (genus Curcurbita), topinambur (genus Helianthus), teff (genus Eragrostis), coconut (genus Cocos), chicory (genus Cichorium), hop (genus Humulus), and fig (genus Ficus).

In one embodiment, the Botanical Powder comprises at least one of, alfalfa (genus Medicago), amaranth (genus Amaranthus), argan (genus Argania), arrowroot, barley (genus Hordeum), lupine (genus Lupinus), oat (genus Avena), rice (genus Oryza), soya (genus Glycine), pea (genus Pisum), and wheat (genus Triticum).

In other embodiments, the Botanical Powder comprises at least one of, baobab (genus Adansonia), hop (genus Humulus), maize (genus Zea), chick pea (genus Cicer), apple (genus Pyrus), citrus (genus citrus), coconut (genus Cocos), alfalfa (genus Medicago), amaranth (genus Amaranthus), argan (genus Argania), arrowroot, barley (genus Hordeum), lupine (genus Lupinus), oat (genus Avena), rice (genus Oryza), soya (genus Glycine), pea (genus Pisum), and wheat (genus Triticum).

In still other embodiments, the Botanical Powder is essentially free of powders of Alpiniae officinari Rhizoma extract, powders of Alpinia, powders of bearberry extract, powders of Centella, powders of cinnamon extract, powders of Citrus medica limonum extract, powders of Cucumis, powders of Echinacea, powders of ginger extract, powders of Glycyrrhiza, powders of grapefruit seed extract, powders of Helianthus annus extract, powders of hydrolyzed oats, powders of Rosmarinus officinalis, powders of Sanguisorba officinalis extract, powders of Sophora flavescens extract, and powders of Terminaliae fructus extract. By "essentially free" it is meant that the Botanical Powder contains less than about 1 wt%, about 0.1 wt% and in some cases 0.01 wt% of these powders.

In another embodiment, the Botanical Powder-Based Delivery System is essentially free of Liposome when the Botanical Powder comprises at least one of Terminaliae fructus extract, Glycyrrhiza, Rosmarinus officinalis, Centella, Echinacea and Alpinia. By "essentially free" it is meant that the Botanical Powder-Based Delivery System contains less than about 1 wt%, about 0.1 wt% and in some cases 0.01wt% of Liposome.

According to one embodiment of this invention, the "Botanical Powder" is comprised of proteins, polysaccharides, lipids, fibers and, optionally water, wherein the amount of protein is about 10 wt% to about 95 wt%, and in some cases from about 25 wt% to about 65 wt%, and in other cases from about 35 wt% to about 55 wt%, and wherein the amount of polysaccharides is about 1 wt% to about 75 wt%, and in some cases from about 5 wt% to about 50 wt%, and in other cases from about 7 wt% to about 20 wt%, and wherein the amount of lipids is about 1 wt% to about 35 wt%, and in some cases from about 5 wt% to about 20 wt%, and in other cases from about 7 wt% to about 15 wt%, and wherein the amount of fibers is about 1 wt% to about 40 wt%, in some cases about 5 wt% to about 35 wt%, and in other cases about 15 wt% to about 35 wt%, and wherein the amount of water is preferably less than about 15 wt%, all based on the total weight of the botanical powder. In one aspect of this embodiment, the botanical powder is from a plant of the genus Lupinus, and in a further aspect is from the species Lupinus albus. In some embodiments, the "Botanical Powder" is comprised of about 1 wt% to about 30 wt% proteins, about 60 wt% to about 90 wt% polysaccharides, about 0.5 wt% to about 10 wt% lipids, about 40 wt% to about 95 wt% fibers, and preferably less than about 15 wt% water, all based on the total weight of the botanical powder.

"Botanical Powders" according to another embodiment of the present invention comprise powders that exhibit partial solubility in water at ambient temperature, and in some cases partial solubility in water at temperatures in the range of 40-60°C, and in other cases partial solubility in water at temperatures in the range of 60-90°C. According to this embodiment of the invention the partial solublity in water of the botanical powder can be measured by a centrifuge test wherein an aqueous dispersion containing 2 wt% of the botanical powder is prepared by mixing the botanical powder in water and heating at a rate of approximately 2 °C per minute to a prescribed temperature (Tₘᵢₓ) for 30 minutes at 350 to 450 rpm (e.g., as described in greater detail in Example 1). The aqueous dispersion is cooled to room temperature, and then the aqueous dispersion is centrifuged at 3000 rpm (1400 g) for 30 minutes by means of an Eppendorf Centrifuge Model 5702. An amount of ethanol equal in weight to the original weight of the centrifuged aqueous dispersion is added to the resultant precipitate, the sample is shaken by hand to wash the precipitate, and the sample is centrifuged another 30 minutes. The precipitate recovered from the ethanol wash is dried to constant weight in a forced air oven at 50 °C. The partial water solubility is then calculated on a scale of 0 (completely water insoluble) to 1 (completely water soluble) as 1 - [dried weight of centrifuge test / initial weight of powder in the dispersion]. In one aspect of this embodiment, when Tₘᵢₓ = 20 °C the partial water solubility of the botanical powder is from about 0.05 to about 0.95, in some cases from about 0.1 to about 0.7, in other cases from about 0.1 to about 0.6, and in still other cases from about 0.15 to about 0.4. In another aspect of this embodiment, when Tₘᵢₓ = 80 °C the partial water solubility of the botanical powder is from about 0.05 to about 0.95, in some cases from about 0.1 to about 0.8, in other cases from about 0.15 to about 0.6, and in yet other cases from about 0.2 to about 0.5.

### Active Compounds

The term "Active Compounds" as used herein means and comprises, without limitation, compounds, substances, molecules, and mixtures that are used in cosmetic and personal care products in order to interact with or protect the skin or hair in a manner that brings about a favorable and intended change in one or more cosmetic skin or hair parameters (e.g. wrinkles, elasticity, for example). Active Compounds may be of synthetic or natural origin, the latter derived from but not limited to any of animal, plant, mineral, or marine sources.

In one embodiment of the invention, Active Compounds include at least one of sunscreen compounds (e.g. zinc oxide, titanium dioxide, octinoxate, octocrylene, ethylhexyl salicylate, oxybenzone); skin whiteners (e.g. salicylic acid, aloesin, ethyl ascorbic acid, arbutin); anti-cellulite compounds; anti-aging compounds (e.g., polypeptides such as Argininie/Lysine, Argininie PCA, Aspergillus/Aspidosperma Quebracho Ferment, botanical actives, Bifida Ferment Lysate, Calophylum Inorhylum seed oil, camellia sinensis extract, ceramides, chlorella vulgaris extract, coriolus versicolor extract, corylus avellana (hazel) seed extract, erythorbic acid, hydrolyzed elastins, hydrolyzed proteins, hydrolyzed soy flour, hydrolyzed peptides, and Vitamins A, E, C, K, B7, and B5 as well as Niacinamide); anti-acne compounds (e.g., salicylic acid, sodium salicylate, benzoyl peroxide); anti-dandruff compounds (e.g., zinc pyrithione); APDO compounds (e.g., aluminum chlorohydrate, aluminum zirconium tetra chlorohydrex); vitamins (e.g., Tocopherol natural, synthetic Tocopherol, synthetic tocopheryl acetate, Retinol, Retinyl palmitate, Retinyl acetate, Provitamin B-5, ascorbic acid, sodium ascorbyl phosphate, Ascorbyl glucoside, Magnesium ascorbyl phosphate); insect repellents; and Coenzymes and Enzymes (e.g., Ubiquinone, Coenzyme Q10); Botanicals (e.g., Aloe vera, Green tea extract, Grape seed extract, Isoflavones, Chamomille/bisabolol, Fennel, Ginko, Ginseng, Guava); Alpha Hydroxy Acids (e.g., Citric acid, Glycolic acid, Lactic acid); Sugar cane extracts; Avena Sativa (Oat) Kernel Protein, and Avocado Sterols, proteins, peptides, copper peptides, fermented biopolymers, beta-glucan, caffeine, Hyaluronic acid and its derivatives like sodium hyaluronate, all by way of example only. In other embodiments, active compounds include at least one of algae chlorella (genus Chlorella), brewer's yeast (genus Saccharomyces) and red yeast grown on rice (genus Monascus). In certain embodiments, an active compound may fall into more than one category and/or be used to accomplish more than one result; as example, alpha hydroxyl acids may be used for smoothing fine lines and surface wrinkles, unblocking and cleansing pores, and for exfoliation, among other functions.

In another embodiment, Active Compounds may also include at least one in vivo Fluorescent Dye. While an in vivo Fluorescent Dye may not interact with or protect skin or hair, it may be used as a model for cosmetic actives to allow for the assessment and prediction of epidermal penetration of cosmetic actives. In vivo Fluorescent Dyes include any fluorescent dye that meets regulatory and/or safety requirements for human in vivo skin penetration studies, and includes Fluorescein Alcon 10%, for example. When used as an Active Compound, the at least one in vivo Fluorescent Dye comprises between about 0.00001 wt% to about 0.1 wt%, and in other cases between about 0.0001 wt% to about 0.01 wt%, and in yet other cases from about 0.0002 wt% to about 0.001 wt%, based on the total weight of the inventive composition.

In certain embodiments of the invention, Active Compounds do not contain, or are essentially free, of sunscreen compounds (e.g. zinc oxide, titanium dioxide, octinoxate, octocrylene, octisalate, cinoxate, ethylhexyl salicylate, avobenzone, oxybenzone, among others). By "essentially free" it is meant that the Active Compound contains less than about 1 wt%, in some cases less than about 0.1 wt%, and in other cases less than 0.01 wt% of sunscreen compounds.

### Cerasome

In one embodiment of the invention, the Botanical Powder is combined with a Delivery System comprising a Cerasome. The term "Cerasome" is denoted to mean an artificially prepared vesicle made of at least one lipid bilayer (lipid membrane), wherein the delivery system comprises naturally-derived or synthetic ceramides and naturally-derived or synthetic glycosphingolipids, which structure can act as a physical reservoir for active ingredients such as for the Botanical Powder.

In a first embodiment, the composition comprises a Botanical Powder and a "Cerasome" delivery system. Compositions of the first embodiment with reduced sedimentation and desirable characteristics for use in Cosmetic or Personal Care compositions are comprised of at least:
i) one or more naturally-derived or synthetic ceramides and/or naturally-derived or synthetic glycosphingolipids in an amount from about 0.1 wt% to about 15 wt%, in some cases from about 0.5 wt% to about 8 wt%, and in other cases from about 1 wt% to about 4 wt%, based on the total weight of the composition,
ii) at least one Botanical Powder in an amount from about 0.1 wt% to about 20 wt%, in some cases from about 0.5 wt% to about 10 wt%, and in other cases from about 1 wt% to about 4 wt%, based on the total weight of the composition,
iii) at least one solvent, comprising at least one alcohol, glycol, or triol,
iv) water in an amount of at least 30 wt%, in some cases at least 50 wt%, and in other cases at least 60 wt%, based on the total weight of the composition,
v) and, optionally, one or more Other Functional Ingredients (defined below).

In some embodiments, the at least one solvent, comprising at least one alcohol, glycol, or triol, is in an amount from about 5 wt% to about 30 wt%, in some cases from about 8 wt% to about 25 wt%, and in other cases from about 10 wt% to about 20 wt%, based on the total weight of the composition. Specific examples of suitable solvents comprise at least one member selected from the group consisting of ethanol, 1,2-propanediol, 1,3-propanediol, 1,3-butylene glycol, and glycerol. In some cases the solvent comprises ethanol in an amount from about 5 to about 30 wt%, and in other cases from about 8 wt% to about 25 wt%.

In certain embodiments, the composition is a Botanical Powder Deliver System that comprises a Botanical Powder and a "Cerasome" Delivery System and wherein the sum of one or more naturally-derived or synthetic ceramides and/or naturally-derived or synthetic glycosphingolipids, at least one Botanical Powder, at least one solvent comprising at least one alcohol, glycol, or triol; and water, is greater than about 75 wt%, in some cases greater than about 90 wt%, in other cases greater than about 95 wt%, and in still other cases greater than about 97.5 wt%, based on the total weight of the composition. If desired, the compositions of the instant invention can be used as an ingredient in a Cosmetic or Personal Care product

In some embodiments of a Botanical Powder and a "Cerasome" delivery system, the Botanical Powder comprises at least one of, alfalfa (genus Medicago), amaranth (genus Amaranthus), argan (genus Argania), arrowroot, barley (genus Hordeum), lupine (genus Lupinus), oat (genus Avena), rice (genus Oryza), soya (genus Glycine), pea (genus Pisum), and wheat (genus Triticum). In yet another embodiment the Botanical Powder is from a plant of the genus Lupinus.

In other embodiments of a Botanical Powder and a "Cerasome" delivery system, the Botanical Powder comprises at least one of, baobab (genus Adansonia), hop (genus Humulus), maize (genus Zea), chick pea (genus Cicer), apple (genus Pyrus), citrus (genus citrus), coconut (genus Cocos), alfalfa (genus Medicago), amaranth (genus Amaranthus), argan (genus Argania), arrowroot, barley (genus Hordeum), lupine (genus Lupinus), oat (genus Avena), rice (genus Oryza), soya (genus Glycine), pea (genus Pisum), and wheat (genus Triticum).

Botanical Powder-Based Delivery Systems that employ "Cerasome" compositions of the invention may be made by mixing together an aqueous phase containing, for example, the at least one Botanical Powder and a ceramide or glycosphingolipids solution according to any suitable method described in the prior art, or known to those skilled in the art. In a first process step, the Botanical Powder is dispersed in water. In certain embodiments, during the first step the Botanical Powder is heated in water at a temperature of about 50 °C to about 90 °C, and in some cases from about 70 °C to about 80 °C, for a period of about 15 minutes to about 120 minutes, and in other cases from about 30 minutes to about 60 minutes, and then in some cases allowed to cool to room temperature. Without being bound to explanation, it is believed that in some cases this step increases the fraction of water soluble material in the Botanical Powder and may thus further reduce sedimentation in the inventive compositions. In a second process step the Ceramides or Glycosphingolipids are dissolved in alcohol, glycol, triol, or a combination thereof, at a temperature of about 30 °C to about 80 °C, and in some cases from about 40 °C to about 60 °C. Finally the aqueous phase containing the Botanical Powder and the Ceramide or Glycosphingolipids solution are mixed together at ambient temperature while stirring and homogenizing by means of a high shear homogenizer, non-limiting examples of which include high shear rotor-stator type homogenizers, high pressure homogenizers and ultrasonic homogenizers, for a period of about 2 minutes to about 90 minutes, and in some cases from about 5 minutes to about 60 minutes.

In other embodiments, Other Functional Ingredients can optionally be incorporated by adding and dissolving them in the aqueous phase, or by adding and dissolving them in the solution containing the Ceramides or Glycosphingolipids, depending on the solubility characteristics of the Other Functional Ingredients, at a temperature of about 20 °C to about 80 °C, and in some cases from about 40 °C to about 60 °C, and in other cases at ambient temperature prior to the mixing and homogenizing step.

The aforementioned at least one lipid bilayer of the "Cerasome" according to the invention may lead to a multilamellar (composed of more than one lipid bilayer) or an unilamellar structure (composed of only one lipid bilayer), wherein unilamellar vesicles are preferred. According to further embodiments of the invention, the average diameter (particle size, herein also referred to as "diameter") of the "Cerasome " is from about 50 nm to about 500 nm, in some cases from about 100 nm to about 400 nm, and in yet other cases from about 150 nm to about 300 nm, as measured by Photon Correlation Spectroscopy (PCS). "Particle Size" or "diameter" refers to the averaged hydrodynamic diameter as measured from diluted aqueous solutions by Photon Correlation Spectroscopy, also referred to as Dynamic Light Scattering. The particle size of "Cerasome" was measured by Photon Correlation Spectroscopy using a MALVERN Zetasizer Nano SZ90 by means of a 5 mW He-Ne laser (633 nm), with a measuring angle of 90° at a temperature of 25 °C +/- 0.5 °C, according to the procedure published in the MALVERN operating manual.

Additionally, the present invention relates to use of an aforementioned method for preparing a composition with reduced sedimentation, as described above, and a composition with reduced sedimentation obtainable or obtained by an aforementioned method, wherein the "Cerasome" have a particle size from about 50 nm to about 500 nm, in some cases from about 100 nm to about 400 nm, and in yet other cases from about 150 nm to about 300 nm, as measured by Photon Correlation Spectroscopy as described previously.

### Liposome I

In one embodiment of the invention the Botanical Powder is combined with a Delivery System comprising a Liposome I. The term "Liposome I" is denoted to mean an artificially prepared vesicle made of at least one lipid bilayer (lipid membrane), wherein the delivery system comprises naturally-derived or synthetic phospholipids of type PL2, which structure can act as a physical reservoir for active ingredients such as for the Botanical Powder. Phospholipids of type PL2 comprise phosphatidylcholine, phosphatidylethanolamine and phosphatidic acid, wherein the content of phosphatidylcholine is from about 10 wt% to about 60 wt%, and in some cases from about 15 wt% to about 45 wt%, and in yet other cases from about 20 wt% to about 35 wt%, based on the total weight of the phospholipids.

In a second embodiment, the composition comprises a Botanical Powder and a "Liposome I" delivery system. Compositions of the second embodiment with reduced sedimentation and desirable characteristics for use in Cosmetic or Personal Care compositions are comprised of at least
i) one or more naturally-derived or synthetic phospholipids of type PL2, in an amount from about 0.1 wt% to about 25 wt%, in some cases from about 1 wt% to about 15 wt%, and in other cases from about 5 wt% to about 10 wt%, based on the total weight of the composition,
ii) at least one Botanical Powder in an amount from about 0.1 wt% to about 20 wt%, in some cases from about 0.5 wt% to about 10 wt%, and in other cases from about 1 wt% to about 4 wt%, based on the total weight of the composition,
iii) at least one solvent, comprising at least one alcohol, glycol or triol,
iv) water in an amount of at least 30 wt%, in some cases at least 50 wt%, and in other cases at least 60 wt%, based on the total weight of the composition,
v) and, optionally, one or more Other Functional Ingredients.

In some embodiments, the at least one solvent, comprising at least one alcohol, glycol, or triol, is in an amount from about 5 wt% to about 50 wt%, in some cases from about 8 wt% to about 35 wt%, and in other cases from about 10 wt% to about 25 wt%, based on the total weight of the composition. In certain embodiments of inventive compositions comprising a "Liposome I", the solvent is selected from one or more of ethanol, 1,2-propanediol, 1,3-propanediol, 1,3-butylene glycol, and glycerol. Wherein the composition comprises ethanol, in some embodiments the amount is from about 5 wt% to about 15 wt%, based on the total weight of the composition. Wherein the composition comprises 1,2-propanediol or 1,3-propanediol, in some embodiments the amount is from about 10 wt% to about 35 wt%, and in other cases from about 15 wt% to about 25 wt%, based on the total weight of the composition. Wherein the composition comprises 1,3-butylene glycol, in some embodiments the amount is from about 5 wt% to about 30 wt%, and in other cases from about 10 wt% to about 25 wt%, based on the total weight of the composition. Wherein the composition comprises glycerol, in some embodiments the amount is from about 15 wt% to about 50 wt%, and in other cases from about 15 wt% to about 35 wt%, based on the total weight of the composition. While any one or more of ethanol, 1,2-propanediol, 1,3-propanediol, 1,3-butylene glycol, or glycerol can provide compositions with reduced sedimentation and suitable for use in personal care, consumer acceptance of ethanol in skin care applications, particularly in face care applications has decreased in recent years, and similarly an increasing number of manufacturers of cosmetic or personal care products prefer to not use 1,2-propanediol or 1,3-propanediol as an ingredient. In some embodiments, the selection of alcohol, glycol or triol is from a plant-based or renewable source.

In certain embodiments, the composition is Botanical Powder Deliver System that comprises a Botanical Powder and a "Liposome I" Delivery System and wherein the sum of one or more naturally-derived or synthetic phospholipids of type PL2, at least one Botanical Powder, at least one solvent, comprising at least one alcohol, glycol, or triol; and water, is greater than about 75 wt%, in some cases greater than about 90 wt%, in other cases greater than about 95 wt%, and in still other cases greater than about 97.5 wt%, based on the total weight of the composition. If desired, the compositions of the instant invention can be used as an ingredient in a Cosmetic or Personal Care product.

In some embodiments of a Botanical Powder and a "Liposome I" delivery system, the Botanical Powder comprises at least one of, alfalfa (genus Medicago), amaranth (genus Amaranthus), argan (genus Argania), arrowroot, barley (genus Hordeum), lupine (genus Lupinus), oat (genus Avena), rice (genus Oryza), soya (genus Glycine), pea (genus Pisum), and wheat (genus Triticum). In yet another embodiment the Botanical Powder is from a plant of the genus Lupinus.

In other embodiments of a Botanical Powder and a "Liposome I" Delivery System, the Botanical Powder comprises at least one of, baobab (genus Adansonia), hop (genus Humulus), maize (genus Zea), chick pea (genus Cicer), apple (genus Pyrus), citrus (genus citrus), coconut (genus Cocos), alfalfa (genus Medicago), amaranth (genus Amaranthus), argan (genus Argania), arrowroot, barley (genus Hordeum), lupine (genus Lupinus), oat (genus Avena), rice (genus Oryza), soya (genus Glycine), pea (genus Pisum), and wheat (genus Triticum).

A Botanical Powder-Based Delivery System comprising "Liposome I" compositions of the invention may be made by mixing together an aqueous phase containing, for example, the at least one Botanical Powder and a solution containing Phospholipids of type PL2 according to any suitable method described in the prior art, or known to those skilled in the art. In a first process step, the Botanical Powder is dispersed in water. In certain embodiments, during the first step the Botanical Powder is heated in water at a temperature of about 50 °C to about 90 °C, and in some cases from about 70 °C to about 80 °C, for a period of about 15 minutes to about 120 minutes, and in other cases from about 30 minutes to about 60 minutes, and then in some cases allowed to cool to room temperature. Without being bound to explanation, it is believed that in some cases this step increases the fraction of water soluble material in the Botanical Powder and may thus further reduce sedimentation in the inventive compositions. In a second process step, a portion of the Phospholipids of type PL2 are optionally mixed into the aqueous phase and homogenized by means of a high shear homogenizer, the optional portion from about 0 to about 99%, and in some cases from about 0 to about 90%, and in other cases about 0 to about 50%, of the total amount of Phospholipids of type PL2 in the composition. In a third process step, the balance of the Phospholipids of type PL2 are dissolved in alcohol, glycol, triol, or a combination thereof, at a temperature of about 20 °C to about 80 °C, and in some cases from about 40 °C to about 60 °C and in other cases at ambient temperature. Finally the aqueous phase containing the Botanical Powder and the solution containing Phospholipids of type PL2 dissolved in alcohol, glycol, triol, or a combination thereof are mixed together at ambient temperature while stirring and homogenizing by means of a high shear homogenizer, non-limiting examples of which include high shear rotor-stator type homogenizers, high pressure homogenizers and ultrasonic homogenizers, for a period of about 2 minutes to about 90 minutes, and in some cases from about 5 minutes to about 60 minutes.

In some embodiments, the membrane-forming lipids of "Liposome I" may optionally comprise further membrane stabilizing components such as cholesterol and/or phytosterol.

In other embodiments, Other Functional Ingredients can optionally be incorporated by adding and dissolving them in the aqueous phase, or by adding and dissolving them in the solution containing Phospholipids of type PL2, depending on the solubility characteristics of the Other Functional Ingredients, at a temperature of about 20 °C to about 80 °C, and in some cases from about 40 °C to about 60 °C, and in other cases at ambient temperature prior to the mixing and homogenizing step.

The aforementioned at least one lipid bilayer of the "Liposome I" according to the invention may lead to a multilamellar or an unilamellar structure, wherein unilamellar vesicles are preferred. According to further embodiments of the invention, the particle size of the "Liposome I" is from about 50 nm to about 600 nm, in some cases from about 150 nm to about 500 nm, and in yet other cases from about 200 nm to about 450 nm, as measured by Photon Correlation Spectroscopy as described previously.

Additionally, the present invention relates to use of an aforementioned method for preparing a composition with reduced sedimentation, as described above, and a composition with reduced sedimentation obtainable or obtained by an aforementioned method, wherein the "Liposome I" have a particle size from about 50 nm to about 600 nm, in some cases from about 150 nm to about 500 nm, and in yet other cases from about 200 nm to about 450 nm, as measured by Photon Correlation Spectroscopy as described previously.

### Liposome II

In another embodiment of the invention, a Botanical Powder is combined with a Delivery System comprising a Liposome II. The term "Liposome II" is denoted to mean an artificially prepared vesicle made of at least one lipid bilayer (lipid membrane), wherein the delivery system comprises naturally-derived or synthetic phospholipids of type PL1, which structure can act as a physical reservoir for active ingredients such as for the Botanical Powder. Phospholipids of type PL1 comprise phosphatidylcholine, phosphatidylethanolamine and phosphatidic acid, wherein the amount of phosphatidylcholine is from about 70 wt% to about 97 wt%, and in some cases from about 73 wt% to about 95 wt%, based on the total weight of the phospholipids, and wherein in some cases the amount of phosphatidylethanolamine is less than about 7 wt%, based on the total weight of the phospholipids, and wherein in some cases the amount of phosphatidic acid is less than about 8 wt%, based on the total weight of the phospholipids.

In a third embodiment, the composition comprises at least one ingredient selected from a Botanical Powder and an active compound, and a "Liposome II" delivery system. Compositions of the third embodiment with reduced sedimentation (and in some cases improved epidermal delivery) and desirable characteristics for use in Cosmetic or Personal Care compositions are comprised of at least
i) one or more naturally-derived or synthetic phospholipids of type PL1, in an amount from about 1 wt% to about 25 wt%, in some cases from about 5 wt% to about 15 wt%, and in other cases from about 7 wt% to about 12 wt%, based on the total weight of the composition,
ii) at least one ingredient selected from at least one Botanical Powder and at least one active compound in an amount from about 0.1 wt% to about 20 wt%, in some cases from about 0.5 wt% to about 10 wt%, and in other cases from about 1 wt% to about 4 wt%, and in yet other cases from about 0.00001 wt% to about 0.1 wt%, based on the total weight of the composition,
iii) at least one solvent, comprising at least one alcohol, glycol or triol,
iv) water in an amount of at least 30 wt%, in some cases at least 50 wt%, and in other cases at least 60 wt%, based on the total weight of the composition,
v) and, optionally, one or more Other Functional Ingredients.

In certain embodiments, the composition consists essentially of ingredients i) through iv) above, that is the composition consists essentially of phospholipids of type PL1, Botanical Powders, active compounds, alcohols, glycols, triols and water. By consists essentially of it is meant that the sum of phospholipids of type PL1, Botanical Powders, active compounds, alcohols, glycols, triols and water is greater than 90 wt%, in some cases greater than 95 wt%, and in still other cases greater than 97.5 wt%, based on the total weight of the composition. If desired, the compositions of the instant invention can be used as an ingredient in a cosmetic or personal care product.

In some embodiments, the at least one solvent, comprising at least one alcohol, glycol, or triol, is in an amount from about 5 wt% to about 50 wt%, in some cases from about 8 wt% to about 40 wt%, and in other cases from about 10 wt% to about 35 wt%, based on the total weight of the composition. Examples of suitable solvents comprise at least one member selected from the group consisting of ethanol, 1,2-propanediol, 1,3-propanediol, 1,3-butylene glycol, and glycerol. Wherein the solvent comprises at least one selected solvent from amongst ethanol, 1,2-propanediol, 1,3-propanediol, 1,3-butylene glycol, and glycerol, in some embodiments the amount of the selected solvent is from about 5 wt% to about 25 wt%, based on the total weight of the composition. In other embodiments the at least one solvent in an amount from about 10 wt% to about 40 wt% comprises ethanol in an amount from about 10 wt% to about 20 wt% and a second solvent selected from 1,2-propanediol, 1,3-propanediol, 1,3-butylene glycol, or glycerol, and in some cases the second solvent is 1,3-butylene glycol or glycerol.

In some embodiments of a Botanical Powder and a "Liposome II" delivery system, the Botanical Powder comprises at least one of, alfalfa (genus Medicago), amaranth (genus Amaranthus), argan (genus Argania), arrowroot, barley (genus Hordeum), lupine (genus Lupinus), oat (genus Avena), rice (genus Oryza), soya (genus Glycine), pea (genus Pisum), and wheat (genus Triticum). In yet another embodiment the Botanical Powder is from a plant of the genus Lupinus.

In other embodiments of a Botanical Powder and a "Liposome II" Delivery System, the Botanical Powder comprises at least one of, baobab (genus Adansonia), hop (genus Humulus), maize (genus Zea), chick pea (genus Cicer), apple (genus Pyrus), citrus (genus citrus), coconut (genus Cocos), alfalfa (genus Medicago), amaranth (genus Amaranthus), argan (genus Argania), arrowroot, barley (genus Hordeum), lupine (genus Lupinus), oat (genus Avena), rice (genus Oryza), soya (genus Glycine), pea (genus Pisum), and wheat (genus Triticum).

A Botanical Powder-Based Delivery System comprising "Liposome II" compositions of the invention may be made by mixing together an aqueous phase containing, for example, the at least one Botanical Powder and a solution containing Phospholipids of type PL1 according to any suitable method described in the prior art, or known to those skilled in the art. In a first process step, the Botanical Powder is dispersed in water. In certain embodiments, during the first step the Botanical Powder is heated in water at a temperature of about 50 °C to about 90 °C, and in some cases from about 70 °C to about 80 °C, for a period of about 15 minutes to about 120 minutes, and in other cases from about 30 minutes to about 60 minutes, and then in some cases allowed to cool to room temperature. Without being bound to explanation, it is believed that in some cases this step increases the fraction of water soluble material in the Botanical Powder and may thus further reduce sedimentation in the inventive compositions. In a second process step the Phospholipids of type PL1 are dissolved in alcohol, glycol, triol, or a combination thereof, at a temperature of about 20 °C to about 80 °C, and in some cases from about 40 °C to about 60 °C and in other cases at ambient temperature. Finally the aqueous phase containing the Botanical Powder and the solution containing Phospholipids of type PL1 are mixed together at ambient temperature while stirring and homogenizing by means of a high shear homogenizer, non-limiting examples of which include high shear rotor-stator type homogenizers, high pressure homogenizers and ultrasonic homogenizers, for a period of about 2 minutes to about 90 minutes, and in some cases from about 5 minutes to about 60 minutes.

During the preparation of inventive compositions, glycols or triols may also be added to the aqueous phase prior to mixing with Phospholipids of type PL1, or to a solution containing Phospholipids of type PL1. Depending on their solubility, active compounds, if included in the composition, may be added to the aqueous phase or to the solution containing Phospholipids. In certain embodiments, the aqueous phase and the Phospholipids of type PL1 are first mixed together while stirring and homogenizing by means of a high shear homogenizer at a temperature of about 50 °C to about 90 °C, and in some cases from about 60 °C to about 85 °C, and in other cases from about 70 °C to about 80 °C, and in yet other cases at an ambient temperature of about 15 °C to about 25 °C. In other embodiments, a solution containing Phospholipids of type PL1 is first mixed together while stirring, and then added to the aqueous phase while stirring and homogenizing by means of a high shear homogenizer at a temperature of about 50 °C to about 90 °C, and in some cases from about 60 °C to about 85 °C, and in other cases from about 70 °C to about 80 °C, and in yet other cases at an ambient temperature of about 15 °C to about 25 °C. In some embodiments, the stirring and homogenizing by means of a high shear homogenizer is followed by high pressure homogenization at a pressure of about 200 bar to about 1000 bar, and in some cases from about 300 bar to about 600 bar, for a period of about 5 to about 60 minutes.

In some embodiments, the membrane-forming lipids of "Liposome II" may optionally comprise further membrane stabilizing components such as cholesterol and/or phytosterol.

In other embodiments, Other Functional Ingredients can optionally be incorporated by adding and dissolving them in the aqueous phase, or by adding and dissolving them in the solution containing Phospholipids of type PL1, depending on the solubility characteristics of the Other Functional Ingredients, at a temperature of about 20 °C to about 80 °C, and in some cases from about 40 °C to about 60 °C, and in other cases at ambient temperature prior to the mixing and homogenizing step.

In yet another embodiment, the compositions allow for targeted topical epidermal delivery of active compounds (as determined by 5D-IVT penetration studies the procedure of which is described in greater detail below), offer desirable characteristics for use in Cosmetic or Personal Care compositions, and are comprised of at least
i) one or more naturally-derived or synthetic phospholipids of type PL1, wherein the amount of phosphatidylcholine is from about 70 wt% to about 97 wt%, in some cases from about 73 wt% to about 95 wt%, in other cases from about 73 wt% to about 79 wt%, in yet other cases from about 85 wt% to about 95 wt%, and in still other cases from about 95 wt% to about 97 wt%, based on the total weight of phospholipids, in an amount from about 1 wt% to about 15 wt%, in some cases from about 1 wt% to about 4 wt%, in other cases from about 2 wt% to about 3 wt%, in yet other cases from about 4 wt% to about 7 wt%, and still other cases from about 7 wt% to about 12 wt%, based on the total weight of the composition,
ii) at least one ingredient selected from at least one Botanical Powder and at least one active compound in an amount from about 0.1 wt% to about 35 wt%, in some cases from about 0.2 wt% to about 20 wt%, in other cases from about 0.5 wt% to about 10 wt%, and in still other cases from about 1 wt% to about 4 wt%, and in yet other cases from about 0.00001 wt% to about 0.1 wt%, based on the total weight of the composition,
iii) at least one solvent, comprising at least one glycol or triol, in an amount from about 5 wt% to about 50 wt%, in some cases from about 10 wt% to about 20 wt%, and in other cases from about 20.5 wt% to about 50 wt%, and in yet other cases from about 30 wt% to about 60 wt%, based on the total weight of the composition,
iv) water in an amount of at least 30 wt%, in some cases at least 50 wt%, and in other cases at least 60 wt%, based on the total weight of the composition,
v) and, optionally, one or more Other Functional Ingredients.

In certain embodiments, the at least one solvent comprising at least one glycol or triol may be essentially free of ethanol. By essentially free it is meant that the total composition contains less than 1 wt% ethanol, in some cases less than 0.5 wt% ethanol, in other cases less than 0.2 wt% ethanol, and in yet other cases less than 0.1 wt% ethanol, based on the total weight of the composition.

In other embodiments, the at least one solvent comprising at least one glycol or triol further comprises at least one alcohol in an amount from about 0.001 wt% to about 10 wt%, in further cases from about 0.001 wt% to about 7.5 wt%, in yet further cases from about 0.001 wt% to about 4 wt%, in some cases from about 0.05 wt% to about 3.5 wt%, in other cases from about 0.05 to about 1 wt%, in yet other cases from about 1 wt% to about 4 wt%, and in still other cases from about 2.5 wt% to about 3.5 wt%, based on the total weight of the composition. In some embodiments, the alcohol comprises phenethyl alcohol.

In certain embodiments, the composition consists essentially of ingredients i) through iv) above, that is the composition consists essentially of phospholipids of type PL1, Botanical Powders, active compounds, glycols, triols. alcohols and water. By consists essentially of it is meant that the sum of phospholipids of type PL1, Botanical Powders, active compounds, glycols, triols, alcohols and water is greater than 90 wt%, in some cases greater than 95 wt%, and in still other cases greater than 97.5 wt%, based on the total weight of the composition.

In another embodiment where the compositions allow for targeted topical epidermal delivery of active compounds (as determined by 5D-IVT penetration studies), and offer desirable characteristics for use in Cosmetic or Personal Care compositions, the one or more naturally-derived or synthetic phospholipids of type PL1 is in an amount from about 2 wt% to about 3 wt%, the at least one solvent comprises at least one triol, in an amount from about 10 wt% to about 20 wt%, and optionally at least one alcohol in an amount from about 0.001 wt% to about 10 wt%, in further cases from about 0.001 wt% to about 7.5 wt%, in yet further cases from about 0.001 wt% to about 4 wt%, in some cases from about 0.05 wt% to about 3.5 wt%, in other cases from about 0.05 to about 1 wt%, in yet other cases from about 1 wt% to about 4 wt%, and in still other cases from about 2.5 wt% to about 3.5 wt%, and water in an amount of at least 60 wt%, all based on the total weight of the composition. In one embodiment, the at least one triol comprises glycerol and the at least one alcohol comprises ethanol.

In another embodiment where the compositions allow for targeted topical epidermal delivery of active compounds (as determined by 5D-IVT penetration studies), and offer desirable characteristics for use in Cosmetic or Personal Care compositions, the one or more naturally-derived or synthetic phospholipids of type PL1 is in an amount from about 2 wt% to about 3 wt%, the at least one solvent comprises at least one triol, in an amount from about 20.5 wt% to about 50 wt%, and optionally at least one alcohol in an amount from about 0.001 wt% to about 10 wt%, in further cases from about 0.001 wt% to about 7.5 wt%, in yet further cases from about 0.001 wt% to about 4 wt%, in some cases from about 0.001 wt% to about 1 wt%, in other cases from about 0.05 to about 3.5 wt%, in yet other cases from about 1 wt% to about 4 wt%, and in still other cases from about 2.5 wt% to about 3.5 wt%, and water in an amount of at least 40 wt%, all based on the total weight of the composition. In one embodiment, the at least one triol comprises glycerol and the at least one alcohol comprises ethanol.

In another embodiment where the compositions allow for targeted topical epidermal delivery of active compounds (as determined by 5D-IVT penetration studies), and offer desirable characteristics for use in Cosmetic or Personal Care compositions, the one or more naturally-derived or synthetic phospholipids of type PL1 is in an amount from about 4 wt% to about 7 wt%, the at least one solvent comprises at least one triol, in an amount from about 10 wt% to about 20 wt%, and optionally at least one alcohol in an amount from about 0.001 wt% to about 10 wt%, in further cases from about 0.001 wt% to about 7.5 wt%, in yet further cases from about 0.001 wt% to about 4 wt%, in some cases from about 0.001 wt% to about 1 wt%, in other cases from about 0.05 to about 3.5 wt%, in yet other cases from about 1 wt% to about 4 wt%, and in still other cases from about 2.5 wt% to about 3.5 wt%, and water in an amount of at least 50 wt%, all based on the total weight of the composition. In one embodiment, the at least one triol comprises glycerol and the at least one alcohol comprises ethanol.

In another embodiment where the compositions allow for targeted topical epidermal delivery of active compounds (as determined by 5D-IVT penetration studies), and offer desirable characteristics for use in Cosmetic or Personal Care compositions, the one or more naturally-derived or synthetic phospholipids of type PL1 is in an amount from about 4 wt% to about 7 wt%, the at least one solvent comprises at least one triol, in an amount from about 20.5 wt% to about 50 wt%, and optionally at least one alcohol in an amount from about 0.001 wt% to about 10 wt%, in further cases from about 0.001 wt% to about 7.5 wt%, in yet further cases from about 0.001 wt% to about 4 wt%, in some cases from about 0.001 wt% to about 1 wt%, in other cases from about 0.05 to about 3.5 wt%, in yet other cases from about 1 wt% to about 4 wt%, and in still other cases from about 2.5 wt% to about 3.5 wt%, and water in an amount of at least 30 wt%, all based on the total weight of the composition. In one embodiment, the at least one triol comprises glycerol and the at least one alcohol comprises ethanol.

In another embodiment,the inventive compositions allow for targeted topical epidermal delivery of active compounds (as determined by 5D-IVT penetration studies), and offer desirable characteristics for use in Cosmetic or Personal Care compositions. The composition comprises one or more naturally-derived or synthetic phospholipids of type PL1 is in an amount from about 4 wt% to about 7 wt%, the at least one solvent comprises at least one triol, in an amount from about 30 wt% to about 60 wt%, and optionally at least one alcohol in an amount from about 0.001 wt% to about 10 wt%, in further cases from about 0.001 wt% to about 7.5 wt%, in yet further cases from about 0.001 wt% to about 4 wt%, in some cases from about 0.001 wt% to about 1 wt%, in other cases from about 0.05 to about 3.5 wt%, in yet other cases from about 1 wt% to about 4 wt%, and in still other cases from about 2.5 wt% to about 3.5 wt%, and water in an amount of at least 30 wt%, all based on the total weight of the composition. In one embodiment, the at least one triol comprises glycerol and the at least one alcohol comprises ethanol.

In another embodiment where the compositions allow for targeted topical epidermal delivery of active compounds (as determined by 5D-IVT penetration studies), and offer desirable characteristics for use in Cosmetic or Personal Care compositions, the one or more naturally-derived or synthetic phospholipids of type PL1 is in an amount from about 7 wt% to about 12 wt%, the at least one solvent comprises at least one triol, in an amount from about 20.5 wt% to about 50 wt%, and optionally at least one alcohol in an amount from about 0.001 wt% to about 10 wt%, in further cases from about 0.001 wt% to about 7.5 wt%, in yet further cases from about 0.001 wt% to about 4 wt%, in some cases from about 0.001 wt% to about 1 wt%, in other cases from about 0.05 to about 3.5 wt%, in yet other cases from about 1 wt% to about 4 wt%, and in still other cases from about 2.5 wt% to about 3.5 wt%, and water in an amount of at least 30 wt%, all based on the total weight of the composition. In one embodiment, the at least one triol comprises glycerol and the at least one alcohol comprises ethanol.

In another embodiment, the inventive compositions allow for targeted topical epidermal delivery of active compounds (as determined by 5D-IVT penetration studies), and offer desirable characteristics for use in Cosmetic or Personal Care compositions. The composition comprisesone or more naturally-derived or synthetic phospholipids of type PL1 is in an amount from about 7 wt% to about 12 wt%, the at least one solvent comprises at least one triol, in an amount from about 30 wt% to about 60 wt%, and optionally at least one alcohol in an amount from about 0.001 wt% to about 10 wt%, in further cases from about 0.001 wt% to about 7.5 wt%, in yet further cases from about 0.001 wt% to about 4 wt%, in some cases from about 0.001 wt% to about 1 wt%, in other cases from about 0.05 to about 3.5 wt%, in yet other cases from about 1 wt% to about 4 wt%, and in still other cases from about 2.5 wt% to about 3.5 wt%, and water in an amount of at least 20 wt%, all based on the total weight of the composition. In one embodiment, the at least one triol comprises glycerol and the at least one alcohol comprises ethanol.

In another embodiment where the compositions allow for targeted topical epidermal delivery of active compounds (as determined by 5D-IVT penetration studies), and offer desirable characteristics for use in Cosmetic or Personal Care compositions, the one or more naturally-derived or synthetic phospholipids of type PL1 is in an amount from about 4 wt% to about 7 wt%, the at least one solvent comprises a glycol, in an amount from about 10 wt% to about 35 wt%, in some cases from about 10 wt% to about 20 wt%, and optionally at least one alcohol in an amount from about 0.001 wt% to about 4 wt%, in some cases from about 0.001 wt% to about 1 wt%, in other cases from about 0.05 to about 3.5 wt%, in yet other cases from about 1 wt% to about 4 wt%, and in still other cases from about 2.5 wt% to about 3.5 wt%, and water in an amount of at least 50 wt%, and in some cases at least 30 wt%, all based on the total weight of the composition. In one embodiment, the at least one alcohol comprises ethanol. In another embodiment, the at least one glycol is selected from 1,3-propanediol, 1,3-butylene glyol and 1,2-pentylene glycol.

In another embodiment where the compositions allow for targeted topical epidermal delivery of active compounds (as determined by 5D-IVT penetration studies), and offer desirable characteristics for use in Cosmetic or Personal Care compositions, the one or more naturally-derived or synthetic phospholipids of type PL1 is in an amount from about 4 wt% to about 7 wt%, the at least one solvent comprises a glycol, in an amount from about 20.5 wt% to about 35 wt%, and optionally at least one alcohol in an amount from about 0.001 wt% to about 4 wt%, in some cases from about 0.001 wt% to about 1 wt%, in other cases from about 0.05 to about 3.5 wt%, in yet other cases from about 1 wt% to about 4 wt%, and in still other cases from about 2.5 wt% to about 3.5 wt%, and water in an amount of at least 30 wt%, all based on the total weight of the composition. In one embodiment, the at least one alcohol comprises ethanol. In another embodiment, the at least one glycol is selected from 1,3-propanediol, 1,3-butylene glyol and 1,2-pentylene glycol.

In another embodiment where the compositions allow for targeted topical epidermal delivery of active compounds (as determined by 5D-IVT penetration studies), and offer desirable characteristics for use in Cosmetic or Personal Care compositions, the one or more naturally-derived or synthetic phospholipids of type PL1 is in an amount from about 7 wt% to about 12 wt%, the at least one solvent comprises a glycol, in an amount from about 10 wt% to about 20 wt%, and optionally at least one alcohol in an amount from about 0.001 wt% to about 4 wt%, in some cases from about 0.001 wt% to about 1 wt%, in other cases from about 0.05 to about 3.5 wt%, in yet other cases from about 1 wt% to about 4 wt%, and in still other cases from about 2.5 wt% to about 3.5 wt%, and water in an amount of at least 50 wt%, all based on the total weight of the composition. In one embodiment, the at least one alcohol comprises ethanol. In another embodiment, the at least one glycol is selected from 1,3-propanediol, 1,3-butylene glyol and 1,2-pentylene glycol.

In another embodiment where the compositions allow for targeted topical epidermal delivery of active compounds (as determined by 5D-IVT penetration studies), and offer desirable characteristics for use in Cosmetic or Personal Care compositions, the one or more naturally-derived or synthetic phospholipids of type PL1 is in an amount from about 7 wt% to about 12 wt%, the at least one solvent comprises a glycol, in an amount from about 20.5 wt% to about 35 wt%, and optionally at least one alcohol in an amount from about 0.001 wt% to about 4 wt%, in some cases from about 0.001 wt% to about 1 wt%, in other cases from about 0.05 to about 3.5 wt%, in yet other cases from about 1 wt% to about 4 wt%, and in still other cases from about 2.5 wt% to about 3.5 wt%, and water in an amount of at least 30 wt%, all based on the total weight of the composition. In one embodiment, the at least one alcohol comprises ethanol. In another embodiment, the at least one glycol is selected from 1,3-propanediol, 1,3-butylene glyol and 1,2-pentylene glycol.

In certain embodiments where the compositions allow for targeted topical epidermal delivery of active compounds (as determined by 5D-IVT penetration studies), and offer desirable characteristics for use in Cosmetic or Personal Care compositions, the inventive composition and embodiments disclosed herein are essentially free of botanical ingredients obtained from lupine (genus Lupinus), where essentially free means less than 0.1 wt%, in some cases less than 0.05 wt%, in other cases less than 0.01 wt%, and in yet other cases less than 0.001 wt%, based on the total weight of the composition.

The aforementioned at least one lipid bilayer of the "Liposome II" according to the invention may lead to a multilamellar or an unilamellar structure, wherein unilamellar vesicles are preferred. According to further embodiments of the invention, the particle size of the "Liposome II" is from about 50 nm to about 600 nm, in some cases from about 100 nm to about 450 nm, and in yet other cases from about 150 nm to about 350 nm, as measured by Photon Correlation Spectroscopy as described previously.

Additionally, the present invention relates to use of an aforementioned method for preparing a composition with reduced sedimentation, as described above, and a composition with reduced sedimentation obtainable or obtained by an aforementioned method, wherein the "Liposome II" have a particle size from about 50 nm to about 600 nm, in some cases from about 100 nm to about 450 nm, and in yet other cases from about 150 nm to about 350 nm, as measured by Photon Correlation Spectroscopy as described previously.

### Ceramides and Glycosphingolipids

Within the context of the present invention the term "ceramide" and "glycosphingolipid" refers to complex lipids with sphingosine or a similar base as the basic structure, so-called "Sphingolipids". They are important components of plant and animal cell membranes and contain three characteristic components: one molecule of fatty acid, one molecule of sphingosine or sphingosine derivative and a polar (head) group, which can sometimes be very large and complex. Sphingosine is one of about 30 long chain aminoalcohols which are found in different types of sphingolipids. In mammals, sphingosine (4-sphingenine) and dihydrosphingosine (sphinganine) are the most usual bases of sphingolipids; in higher plants and yeasts, it is phytosphingosine (4-hydroxysphinganine) and in marine invertebrates, they are doubly unsaturated bases such as 4,8-sphingadins. The sphingosine base is bonded to a long chain unsaturated or monounsaturated fatty acid containing 18 to 26 carbon atoms via an amide bond in its amino group. This compound, which contains two non-polar chains, is ceramide, the characteristic basic structure of all sphingolipids. Unless expressly otherwise given, in the context of this application the term "ceramide" will have the same meaning as the term "sphingolipid".

The various derivatives of sphingolipids or ceramides can be divided further into groups depending on their behaviour and structural chemical features. The most usual sphingolipids in the tissues of higher animals are sphingomyelins, which contain phosphorylethanolamine or phosphorylcholine as their polar groups. Another group of sphingolipids contains one or more neutral sugars as the polar group; they thus have no electric charge and hence are termed "neutral glycosphingolipids". Cerebrosides are the simplest members of this group; they have only one monosaccharide in [beta]-glycosidic combination with their hydroxyl group as the polar group. D-galactose is present in the cerebrosides of the brain and nervous system; they are thus termed galactocerebrosides. Cerebrosides are also present in small amounts in non neutral tissues; here, they contain mainly D-glucose and hence are termed glucocerebrosides. Sulphate esters of galactocerbrosides are termed sulphatides, which are also present in brain tissue. Cerebrosides and sulphatides contain fatty acids containing 22 to 26 carbon atoms. A common fatty acid in cerebrosides is cerebronic acid.

"Neutral glycosphingolipids" with one disaccharide are termed dihexosides. Tri-and tetra-hexosides are also known. The sugars in these glycosphingolipids are D-glucose, D-galactose, N-acetyl-D-glucosamine and N-acetyl-D-galactosamine. "Neutral glycosphingolipids" are important components of cell surfaces in animal tissues. Their non polar portion is anchored in the double lipid layer of the membrane, while the polar portion projects from the surface.

Another group of sphingolipids is acidic glycosphingolipids, which are termed gangliosides. Their oligosaccharide portion contains one or more sialinic acids. Gangliosides are present in grey matter.

Galactolipids are membrane lipids primarily present in plants. MGDG (monogalactosyldiacylglycerol) and DGDG (digalactosyldiacylglycerol) are the most common galactolipids in higher plants. They are primarily present in plastides and accumulate in particular in the thyalkoids of chloroplasts.

Thus said the embodiment "Cerasome" according to the invention can include the membrane-forming lipids from the group of naturally-derived or synthetic ceramides and naturally-derived or synthetic glycosphingolipids, and more preferably naturally-derived ceramides and naturally-derived glycosphingolipids that are obtained by methods known in the art. In particular naturally-derived ceramides and naturally-derived glycosphingolipids are obtained from botanicals that are rich in ceramides, including but not limited to wheat, by extraction, maceration or percolation of the material with a suitable solvent and, optionally, by partial or complete removal of the solvent. Thus, extracts in accordance with this invention are either so-called solvent-processed fluid extracts or so called dry extracts obtained by evaporation of the whole liquid extract to dryness, e.g. by air drying, spray drying, vacuum oven drying, fluid-bed drying or freeze-drying, and optional washing and/or re-dissolving of this dry extract in at least one suitable solvent. Solvents suitable for extraction, percolation or maceration are known to those experienced in the art. Acetone, chloroform, ethyl acetate, lower alkanols with 1 to 4 carbon atoms, alcohols or a mixture of these and water are particularly suited. Carbon dioxide in fluid or super-critical form and pressurized gases with solvent properties are also suitable as extraction agents.

Within certain embodiments of the invention, "Cerasome" includes membrane-forming lipids comprising ceramides and glycosphingolipids, wherein the amount of ceramides and glycosphingolipids are from about 25 wt% to about 95 wt%, in some cases from about 35 wt% to about 70 wt%, and in other cases from about 40 wt% to about 60 wt%, based on the total weight of all membrane forming lipids.

According to a further embodiment, ceramides may comprise in addition diacylglycosides, wherein the amount of diacylglycosides is from about 20 wt% to about 60 wt%, and in some cases from about 35 wt% to about 45 wt%, based on the total weight of the membrane forming lipids.

### Phospholipids

Within the context of the present invention the term "phospholipid" refers to a lipid or glyceride that contains a phosphate group. Thus the phospholipid may be, for example, lecithin, phosphatidylethanolamine, phosphatidylinositol, phosphatidylserine, diphosphatidyl glycerol (cardiolipin), dilauroyl phosphatidylcholine, dimyristoyl phosphatidylcholine, dipalmitoyl phosphatidylcholine, distearoyl phosphatidylcholine, dioleoyl phosphatidylcholine, dimyristoyl phosphatidylethanolamine, dipalmitoyl phosphatidylethanolamine, dipalmitoyl phosphatidyl glycerol, dimyristoyl phosphatidic acid, dipalmitoyl phosphatide acid, dipalmitoyl phosphatidylserine, dipalmitoyl sphingomyelin, 1-stearoyl-2palmitoyl phosphatidylcholine.

The term "lecithin", within the context of the invention, refers to a naturally occurring or synthetic lecithin, which may be suitably refined. Suitable lecithins include, but are not limited to lecithins derived from soybean, sunflower, or egg. Further suitable lecithins include, but are not limited to dihexanoyl-L-alpha-lecithin, dioctanoyl-L-alpha-lecithin, didecanoyl-L-alpha-lecithin, didodecanoyl-L-alpha-lecithin, ditetradecanoyl-L-alpha-lecithin, dihexadecanoyl-L-alpha-lecithin, dioctadecanoyl-L-alpha-lecithin, dioleoyl-Lalpha-lecithin, dilinoleoyl-L-alpha-lecithin, alpha- palmitoyl. Lecithins are typically mixtures of diglycerides of fatty acids linked to the choline ester of phosphoric acid, and can contain differing amounts of other compounds depending on the method of isolation. Typically, commercial lecithin is a mixture of acetone-insoluble phosphatides. Preferably, the lecithin is obtained from seeds including, but not limited to, soybean, corn, sunflower, rape and lupine, using methods well known in the art.

Soy bean lecithin, obtained as described above, usually comprises phosphatidylcholine, phosphatidylethanolamine and phosphatidic acid as key phospholipid ingredients.

Phospholipids of type PL1 include naturally-derived or synthetic phospholipids selected from among previously described phospholipids, and comprising phosphatidylcholine, phosphatidylethanolamine and phosphatidic acid, wherein the amount of phosphatidylcholine is from about 70 wt% to about 97 wt%, and in some cases from about 73 wt% to about 95 wt%, based on the total weight of the phospholipids, and wherein in some cases the amount of phosphatidylethanolamine is less than about 7 wt%, based on the total weight of the phospholipids, and wherein in some cases the amount of phosphatidic acid is less than about 8 wt%, based on the total weight of the phospholipids.

Phospholipids of type PL2 include naturally-derived or synthetic phospholipids selected from among previously described phospholipids, and comprising phosphatidylcholine, phosphatidylethanolamine and phosphatidic acid, wherein the content of phosphatidylcholine is from about 10 wt% to about 60 wt%, and in some cases from about 15 wt% to about 45 wt%, and in yet other cases from about 20 wt% to about 35 wt%, based on the total weight of the phospholipids.

### Solvents

Embodiments according to the invention include water and solvents from, but not limited to, the group of naturally-derived or synthetic alcohols, naturally-derived or synthetic glycols and naturally-derived or synthetic polyols, including triols, wherein alcohols comprise primary, secondary and tertiary alcohols that are commonly used and accepted in cosmetic formulations and compositions. Alcohols and glycols accepted in cosmetic products include, but are not limited to, ethanol, 1,2-propanediol, 1,3-propanediol, 1,3-butylene glycol, and polyols accepted in cosmetic products include, but are not limited to, glycerol.

According to further embodiments according to the invention the solvents comprising naturally-derived or synthetic alcohols may be combined with naturally-derived or synthetic glycols and/or naturally-derived or synthetic polyols in weight ratios (alcohol: glycol and/or polyol) from about 1 : 0.1 to about 1 : 10, in some cases from about 1 : 0.4 to about 1 : 6, and other cases from about 1 : 0.6 to about 1 : 4, and in yet other cases from about 1 : 1 to about 1 : 2.

### Other Functional Ingredients

Optionally in certain embodiments of the invention the composition according to the invention comprises one or more Other Functional Ingredients that are commonly used in cosmetic formulations and compositions. The term Other Functional Ingredients in connection with the present invention includes substances that may provide benefit to the physical properties of the embodiments and their stability and/or prevent or retard microbial growth. Other Functional Ingredients comprise without limitation chelating agents, preservatives, pH buffers, and Other Additives or Actives (as described below, where many of these same ingredients can also be added to personal care products containing Botanical Powder-Based Delivery Systems). The amount of Other Functional Ingredients typically ranges from about 0.1 wt% to about 25 wt%, in some cases about 0.1 wt% to about 8 wt%, and in other cases about 0.1 wt% to about 2 wt%.

### Chelating Agents

Optionally in certain embodiments of the invention the composition according to the invention comprises at least one chelating agent. According to further embodiments of the foregoing, the at least one preservative is selected from but not limited to the group consisting of ethylenediamintetraacetic acid (EDTA) and its salts e.g. disodium EDTA, nitrilotriacetic acid (NTA) and its salts, cosmetic acceptable phosphates and phosphonates, salts and esters of oxalic acid, tartaric acid, phytic acid, glutamic acid and its salts and preferably N,N- bis(carboxymethyl)glutamic acid and its salts and their blends. In general, the chelating agents may be used in any amount which is effective to prevent cationic destabilization of the compositions.

According to a certain embodiment of the invention, the composition comprises tetrasodium N,N-bis(carboxylatomethyl)-L-glutamate in an amount from about 0.001 wt% to about 1 wt%, in some cases from about 0.01 wt% to about 0.5 wt%, and in other cases from about 0.05 wt% to about 0.3 wt%, based on the total weight of the composition.

### Preservatives

Optionally in certain embodiments of the invention the composition according to the invention comprises at least one preservative. According to further embodiments of the foregoing, the at least one preservative is selected from but not limited to the group consisting of butylparaben; ethylparaben; Isobutylparaben, imidazolidinyl urea; methylparaben , sorbic acid and its salts, O- phenylphenol; propylparaben; quaternium-14; quaternium- 5; sodium dehydroacetate; phenoxyethanol, phenoxyisopropanol, benzyl alcohol, polyaminopropyl biguanide, triethylene glycol, piroctone olamine, benzoic acid and its salts, dehydroacetic acid and its salts, diazolidinyl urea, iodopropynyl butylcarbamate, methylisothiazolinone, glyceryl caprylate, phenylethylalcohol, caprylyl glycol, capryloyl glycine, phenylpropanol, ethylhexylglycerin, ethylpropanediol, and their salts and their blends. In general, the preservatives may be used in any amount which is effective to prevent or retard microbial growth and typically about 0.01 wt% to about 5 wt%, and in some cases from about 0.05 wt% to about 3 wt%, based on the total weight of the composition.

According to a further embodiment of the invention, the composition comprises benzyl alcohol and benzoic acid and dehydroacetic acid, wherein the amount of benzyl alcohol is from about 0.01 wt% to about 3 wt%, in some cases from about 0.1 wt% to about 2 wt%, and in other cases from about 0.5 to about 1.0 wt%, and wherein the amount of benzoic acid is from about 0.0015 wt% to about 0.45 wt%, in some cases from about 0.015 wt% to about 0.3 wt%, and in other cases from about 0.075 wt% to about 0.15 wt%, and wherein the amount of dehydroacetic acid is from about 0.001 wt% to about 0.3 wt%, in some cases from about 0.01 wt% to about 0.2 wt%, and in other cases from about 0.05 wt% to about 0.1 wt%, all based on the total weight of the composition.

In one embodiment, the Botanical Powder-Based Delivery System uses a "Cerasome" Delivery System comprising one or more naturally-derived or synthetic ceramides and/or naturally-derived or synthetic glycosphingolipids in an amount from about 0.5 wt% to about 8 wt%, and typically from about 1 wt% to about 4 wt%, at least one Botanical Powder in an amount from about 0.5 wt% to about 10 wt%, and typically from about 1 wt% to about 4 wt%, at least one alcohol, glycol, or triol in an amount from about 5 wt% to about 30 wt%, and water in an amount at least 50 wt%, all based on the total weight of the composition, and wherein the partial water solubility of the Botanical Powder at 80 °C is from about 0.05 to about 0.95, and in some cases from about 0.1 to about 0.7. In one aspect of this embodiment, the at least one alcohol, glycol, or triol is ethanol in an amount from about 8 wt% to about 25 wt%. In another aspect of this embodiment, the Botanical Powder comprises at least one of, baobab (genus Adansonia), hop (genus Humulus), maize (genus Zea), chick pea (genus Cicer), apple (genus Pyrus), citrus (genus citrus), coconut (genus Cocos), alfalfa (genus Medicago), amaranth (genus Amaranthus), argan (genus Argania), arrowroot, barley (genus Hordeum), lupine (genus Lupinus), oat (genus Avena), rice (genus Oryza), soya (genus Glycine), pea (genus Pisum), and wheat (genus Triticum). In another aspect of this embodiment, the Botanical Powder comprises at least one of, alfalfa (genus Medicago), amaranth (genus Amaranthus), argan (genus Argania), arrowroot, barley (genus Hordeum), lupine (genus Lupinus), oat (genus Avena), rice (genus Oryza), soya (genus Glycine), pea (genus Pisum), and wheat (genus Triticum). In still another aspect of the embodiment, the at least one Botanical Powder is a lupine powder comprising protein in an amount from about 35 wt% to about 60 wt%, polysaccharides in an amount from about 7 wt% to about 15 wt%, lipids in an amount from about 7 wt% to about 15 wt%, and fibers in an amount from about 5 wt% to about 35 wt%, all based on the total weight of the lupine powder. In a further aspect of this embodiment, the composition of any previously presented aspect of this embodiment also comprises the Other Functional Ingredients of benzyl alcohol, benzoic acid, dehydroacetic acid and salts of N,N-bis(carboxymethyl)glutamic acid.

In another embodiment, the Botanical Powder-Based Delivery System uses a "Liposome I" Delivery System comprising one or more naturally-derived or synthetic phospholipids of type PL2 in an amount from about 1 wt% to about 15 wt%, and typically from about 5 wt% to about 10 wt%, at least one Botanical Powder in an amount from about 0.5 wt% to about 10 wt%, and typically from about 1 wt% to about 4 wt%, at least one alcohol, glycol or triol, in an amount from about 5 wt% to about 50 wt%, and typically from about 8 wt% to about 35 wt%, and water in an amount at least 50 wt%, and typically at least 60 wt%, all based on the total weight of the composition, and wherein the phosphatidylcholine content of the phospholipids is from about 15 wt% to about 45 wt%, and in some cases from about 20 wt% to about 35 wt%, based on the total weight of the phospholipids, and wherein the partial water solubility of the Botanical Powder at 80 °C is from about 0.05 to about 0.95, and in some cases from about 0.1 to about 0.7. In one aspect of this embodiment, the Botanical Powder comprises at least one of, alfalfa (genus Medicago), amaranth (genus Amaranthus), argan (genus Argania), arrowroot, barley (genus Hordeum), lupine (genus Lupinus), oat (genus Avena), rice (genus Oryza), soya (genus Glycine), pea (genus Pisum), and wheat (genus Triticum). In another aspect of this embodiment, the Botanical Powder comprises at least one of, baobab (genus Adansonia), hop (genus Humulus), maize (genus Zea), chick pea (genus Cicer), apple (genus Pyrus), citrus (genus citrus), coconut (genus Cocos), alfalfa (genus Medicago), amaranth (genus Amaranthus), argan (genus Argania), arrowroot, barley (genus Hordeum), lupine (genus Lupinus), oat (genus Avena), rice (genus Oryza), soya (genus Glycine), pea (genus Pisum), and wheat (genus Triticum). In another aspect of the embodiment, the at least one Botanical Powder is a lupine powder comprising protein in an amount from about 35 wt% to about 60 wt%, polysaccharides in an amount from about 7 wt% to about 15 wt%, lipids in an amount from about 7 wt% to about 15 wt%, and fibers in an amount from about 5 wt% to about 35 wt%, all based on the total weight of the lupine powder. In one aspect of this embodiment, the solvent comprises glycerol in amount from about 15 wt% to about 50 wt%, and typically from about 15 wt% to about 35 wt%, based on the total weight of the composition. In another aspect of this embodiment, the solvent comprises 1,3-butylene glycol in an amount from about 10 wt% to about 25 wt%, based on the total weight of the composition. In yet another aspect of this embodiment, the solvent comprises 1,2-propanediol or 1,3-propanediol in an amount from about 10 wt% to about 35 wt%, based on the total weight of the composition. In still another aspect of this embodiment, the solvent comprises ethanol in an amount from about 5 wt% to about 15 wt%, based on the total weight of the composition. In a further aspect of this embodiment, the composition of any previously presented aspect of this embodiment also comprises the Other Functional Ingredients of benzyl alcohol, benzoic acid, dehydroacetic acid and salts of N,N-bis(carboxymethyl)glutamic acid.

In another embodiment, the Botanical Powder-Based Delivery System uses a "Liposome II" Delivery System comprising one or more naturally-derived or synthetic phospholipids of type PL1 in an amount from about 1 wt% to about 25 wt%, and typically from about 5 wt% to about 15 wt%, at least one Botanical Powder in an amount from about 0.5 wt% to about 10 wt%, and typically from about 1 wt% to about 4 wt%, at least one alcohol, glycol or triol, in an amount from about 5 wt% to about 50 wt%, and typically from about 10 wt% to about 40 wt%, and water in an amount at least 50 wt%, and typically at least 60 wt%, all based on the total weight of the composition, and wherein wherein the partial water solubility of the Botanical Powder at 80 °C is from about 0.05 to about 0.95, and in some cases from about 0.1 to about 0.7. In one aspect of this embodiment, the Botanical Powder comprises at least one of, alfalfa (genus Medicago), amaranth (genus Amaranthus), argan (genus Argania), arrowroot, barley (genus Hordeum), lupine (genus Lupinus), oat (genus Avena), rice (genus Oryza), soya (genus Glycine), pea (genus Pisum), and wheat (genus Triticum). In another aspect of this embodiment, the Botanical Powder comprises at least one of, baobab (genus Adansonia), hop (genus Humulus), maize (genus Zea), chick pea (genus Cicer), apple (genus Pyrus), citrus (genus citrus), coconut (genus Cocos), alfalfa (genus Medicago), amaranth (genus Amaranthus), argan (genus Argania), arrowroot, barley (genus Hordeum), lupine (genus Lupinus), oat (genus Avena), rice (genus Oryza), soya (genus Glycine), pea (genus Pisum), and wheat (genus Triticum). In another aspect of this embodiment, the at least one Botanical Powder is a lupine powder comprising protein in an amount from about 35 wt% to about 60 wt%, polysaccharides in an amount from about 7 wt% to about 15 wt%, lipids in an amount from about 7 wt% to about 15 wt%, and fibers in an amount from about 5 wt% to about 35 wt%, all based on the total weight of the lupine powder. In one aspect of this embodiment, the solvent comprises glycerol, typically from about 5 wt% to about 25 wt%, based on the total weight of the composition. In another aspect of this embodiment, the solvent comprises 1,3-butylene glycol, typically from about 5 wt% to about 25 wt%, based on the total weight of the composition. In yet another aspect of this embodiment, the solvent comprises ethanol, typically from about 5 wt% to about 25 wt%, based on the total weight of the composition. In a further aspect of this embodiment, the composition of any previously presented aspect of this embodiment also comprises the Other Functional Ingredients of benzyl alcohol, benzoic acid, dehydroacetic acid and salts of N,N-bis(carboxymethyl)glutamic acid.

### Reduced Sedimentation and Measurement Method.

The reduced sedimentation benefit of the inventive compositions can be measured by a centrifuge test through comparison of an inventive composition comprising a Botanical Powder and a Delivery System with an aqueous dispersion of the Botanical Powder having the same content of Botanical Powder and prepared under similar conditions. For example, an inventive composition containing 2 wt% Botanical Powder is prepared by first adding the Botanical Powder to water while stirring at 350 to 450 rpm, heating the mixture at a rate of approximately 2 °C per minute to 80 ± 2 °C while stirring, maintaining the temperature and stirring for 30 minutes after the mixture reached 80 °C, cooling the mixture to room temperature while stirring, and then completing the preparation of the inventive composition following any of the previously described steps for making a "Cerasome", "Liposome I" (e.g., as described in greater detail in Example 2) or "Liposome II". A dispersion of 2 wt% Botanical Powder in water is also prepared by adding the Botanical Powder to water while stirring at 350 to 450 rpm, heating the mixture at a rate of approximately 2 °C per minute to 80 ± 2 °C while stirring, maintaining the temperature and stirring for 30 minutes after the mixture reached 80 °C, and cooling the mixture to room temperature while stirring (e.g., as described in greater detail in Example 1). Both the inventive composition and the aqueous dispersion are then centrifuged at the same time for 30 minutes by means of an Eppendorf Centrifuge Model 5702 at 3000 rpm (1400 g). An amount of ethanol equal in weight to the original weight of the centrifuged samples is added to the resultant precipitates, the samples are shaken by hand to wash the precipitates, and the samples are centrifuged another 30 minutes. The precipitates recovered from the ethanol wash are dried to constant weight in a forced air oven at 50 °C. The reduced sedimentation in percent is calculated as 100 x [dry weight of aqueous disperion precipitate - dry weight of inventive composition precipitate] / [dry weight of aqueous dispersion precipitate]. In some embodiments, the Botanical Powder-Based Delivery System exhibits a "reduced sedimentation" of greater than about 20%, in other cases greater than about 35%, and in yet other cases greater than about 50%.

### Determination of Targeted Topical Epidermal Delivery

In some embodiments of the invention, the compositions allows for targeted topical epidermal delivery of active compounds. Epidermal penetration of active compounds can be performed by a variety of methods known to one skilled in the art, that include ex vivo penetration studies on skin biopsies by means of Franz diffusion cells and subsequent detection of active compounds through any suitable means, in vivo strip testing of skin and subsequent quantification of the active compounds through any suitable means, and in vivo by 5D-IVT. For purposes of this invention, the targeted epidermal delivery of active compounds incorporated within inventive compositions was investigated with in vivo 5D-IVT using a multiphoton tomography instrument manufactured by JenLab (JenLab GmbH, Jena, Germany) at ambient temperatures and atmospheric conditions. An amount of 2 mg/cm² of an inventive composition containing an active compound was non-occlusively applied to the inner forearm. After a period of up to about 5 hours from application of the inventive composition, the treated area was cleaned with a compress that was impregnated with an alcohol based disinfectant and subsequently dried with a clean, dry compress. Fifteen minutes after the cleaning procedure the 5D-IVT probe was adjusted to the treated area on the inner forearm of the panelist and the skin was irradiated with near infrared radiation of a wavelength of 900 nm with a pulse length of 100 fs provided by a Titanium:Sapphire tuneable laser system (Mai Tai, Newport Spectra-Physics, USA). The excitation laser beam was attenuated by the use of a Glan calcite polarizer and scanned by two galvanometric mirrors. After passing a beam expander and collimator, the laser pulses are reflected by a dichroic beam splitter into a 40x oil immersion microscope objective with a numerical aperture of 1.3 (Carl Zeiss Jena GmbH, Jena, Germany). The emitted fluorescence light was transmitted and cleared by an additional shortwave-pass filter (F37-490 BrightLine HC 490/LP and F75-680 Multiphoton-Emitter HC 680/SP, F39-461 BrightLine HC 460/60, F39-390 BrightLine HC 390/40; Semrock Inc., Rochester, NY, USA) and split up in three spectral regions by a set of dichroic beam splitters (F43-031 425DCXR, F33-499 495DCXR; Chroma Technology Corp., Bellows Falls, VT, USA). Three separate photomultipliers (PMTs) provided intensity images of the sample and each PMT was readout by time-correlated single photon counting to calculate wavelength-range-specific fluorescence lifetime values. The data of the PMTs were processed by a high-resolution time-correlated single photon counting imaging module (SPC 830, Becker & Hickl GmbH, Berlin, Germany) in order to monitor and record the in vivo fluorescence image of the human skin. Subsequently the relative dermal penetration of active compounds was determined based on the intensities of the characteristic Fluorescence-lifetime imaging signal. Any of various published modes of operation of 5D-IVT instruments, such as those described in "A comparative study of different instrumental concepts for spectrally and lifetime-resolved multiphoton intravital tomography (5D-IVT) in dermatological applications" (Proceedings of SPIE Vol. 7568), for example, may be suitable to image targeted topical epidermal delivery of active compounds.

In certain embodiments and without wishing to be bound by any theory or explanation, targeted topical epidermal delivery may be established in a 5D-IVT image by a count of discrete domains within the image that are assigned to the active compound. In some cases, the count of discreet domains assigned to the active compound in an image area of 60 µm x 60 µm obtained at a depth of 30 µm beneath the skin surface 5 hours after application of the inventive compound to the skin is greater than about 25, in other cases greater than about 100, and in still other cases greater than about 500. In other embodiments, targeted topical epidermal delivery may be established in a 5D-IVT image by the percentage of area within the image that is assigned to the active compound. In some cases, the percentage of area assigned to the active compound in an image area of 60 µm x 60 µm obtained at a depth of 30 µm beneath the skin surface 5 hours after application of the inventive compound to the skin is greater than about 0.1 %, in other cases greater than about 1%, in still other cases greater than about 5%, and in yet other cases greater than about 10%. In still other embodiments, epidermal delivery of the active compound of an inventive composition is increased relative to the same composition without the phospholipids of type PL1 or at least one glycol or triol, such that the count of discreet domains assigned to the active compound, or the percentage of area assigned to the active compound, for the inventive composition is about 2 times greater, in other cases about 5 times greater, and in still other cases about 25 times greater, than the same composition without the phospholipids of type PL1 or at least one glycol or triol. In yet other embodiments, targeted epidermal delivery may be determined by measurement of the permeation rate of the active compound through human skin by any suitable ex vivo or in vitro method known to the art, particularly through the use of a Franz-type diffusion cell. In some cases, the permeation rate of the active compound through human skin for an inventive composition is greater than about 1.25 times, in other cases greater than about 1.5 times, in still other cases greater than about 3.0 times, and in yet other cases greater than about 10.0 times, the permeation rate of the active compound through human skin for the same composition without the phospholipids of type PL1 or at least one glycol or triol. In some embodiments, targeted topical epidermal delivery of active compounds according to any of the aforementioned embodiments is determined 30 minutes after, in other cases 1 hour after, in still other cases 2 hours after, and in yet other cases 3 hours after, application of the inventive composition to the skin.

### Personal care products and exemplary formulations using Delivery Systems

The Delivery System compositions disclosed herein, including Botanical Powder-Based Delivery System compositions, may be employed as an ingredient in creating a Cosmetic or Personal Care product that provides increased effectiveness for topical application with a moisturizing effect, a barrier effect on the skin, sensory benefits, and as a texturing agent, with such benefits provided solely by the Delivery System or in combination with other cosmetic ingredients. A key aspect includes a cosmetic composition that has a Delivery System composition as a first moisturizing ingredient on its own or with combination of other moisturizing ingredients or actives. Delivery System compositions may also provide delivery enhancement and increase the efficacy of other actives, whether the actives are a component of the Delivery System or an ingredient of the Personal Care product. The Delivery System composition is generally present at about 0.1 to about 20% by weight in such products, in some cases between about 0.5 and about 15% by weight, and in still other cases is present between about 1% and about 10% by weight. The composition can be used with a wide range of personal care products having a base media that can comprise at least one member selected from the group consisting of cosmetic oil (i.e. oils compatible for cosmetic uses), water, alcohol and combinations thereof, all by way of example only.

In certain embodiments of a Personal Care or Cosmetic composition that employ a Delivery System composition as an ingredient, the Personal Care or Cosmetic composition is substantially free of ethanol, where substantially free means less than 0.5 wt% ethanol, in some cases less than 0.25 wt% ethanol, in other cases less than 0.1 wt% ethanol, in yet other cases about 0.005 wt% to about 0.25 wt%, and in still other cases about 0.001 wt% to about 0.09 wt% ethanol, based on the total weight of the Personal Care or Cosmetic composition.

The Delivery System composition can be added to Personal Care products as is, or as dispersion in water or any suitable organic solvent or combination of solvents and/or water.

The Cosmetic composition may include, in accordance with one exemplary embodiment, Delivery System composition that when applied to the skin can provide moisturizing benefits. The Cosmetic composition containing a Delivery System may minimize itching of the skin, and may assist in delivering other cosmetic active effectively to the skin. Additional components may be included in the cosmetic composition in order to achieve various beneficial results when applied to the body or hair of a user. All of the components of the cosmetic composition might be natural components or can be combination of natural and synthetic components. The cosmetic composition can be provided with a number of different components in varying quantities in order to achieve a desired result. By way of example, the cosmetic composition may be provided for use on dry or normal skin, and can be applied to the skin of a user in order to increase skin moisture, look, and/or softness.

The Cosmetic composition can also be used to provide moisturization to hair and improve hair luster, hair strength. Delivery System composition may also act as deeper skin moisturizer by maintaining moisture in the skin by creating a barrier system on the skin surface, and in some embodiments may also impart moisture to deeper layers of the skin. Delivery System composition may provide high hydration to skin by easily absorbing, and might accelerate a natural fresh look to the skin. Delivery System composition may quickly absorb into the skin in a few minutes or less, such that it is no longer on the skin surface, and able to provide moisturizing and delivery of active benefits to the deeper skin layers of the epidermis.

Exemplary cosmetic, toiletry and topical health care type personal care products in which the Delivery System composition may be employed include, without limitation, leave-on personal care products (i.e., products that are left on keratinous substrates after application); rinse-off personal care products (i.e., products that are washed or rinsed from keratinous substrates during or within a few minutes of application); at least one of shampoos; chemical and non-chemical hair curling and hair straightening products; hair style maintaining and hair conditioning products; lotions and creams for nails, hands, feet, face, scalp and/or body; hair dye; face and body makeup; nail care products; astringents; deodorants; antiperspirants; anti-acne; antiaging; depilatories; colognes and perfumes; skin protective creams and lotions (such as sunscreens); skin and body cleansers; skin conditioners; skin toners; skin firming compositions; skin tanning and lightening compositions; liquid soaps; bar soaps; bath products; shaving products; and oral hygiene products (such as toothpastes, oral suspensions, and mouth care products), any or the foregoing of which may additionally contain pharmaceutical, phytopharmaceutical and/or neutraceutical ingredients.

The form of such Personal Care products includes, without limitation, a liquid, gel, spray, emulsion (such as lotions and creams), shampoo, pomade, foam, tablet, stick (such as lip care products), makeup, suppositories, among others, any of which can be applied to the skin or hair and which typically are designed to remain in contact there with until removed, such as by rinsing with water or washing with shampoo or soap. Other forms could be gels that can be soft, stiff, or squeezable. Emulsions can be oil-in-water, water-in-oil, water-and-silicone, or multiphase. Sprays can be non-pressurized aerosols delivered from manually pumped finger-actuated sprayers or can be pressurized aerosols. In some embodiments, exemplary Delivery System composition are formulated in aerosol compositions such as mousse, spray, or foam forming formulation, where a chemical or gaseous propellant is used.

Personal Care products containing a Delivery System, can be broadly categorized as gel or emulsions products, water-in-oil emulsions, oil-in-water emulsions, or aqueous or alcohol based systems.

Delivery System composition can be effective with a broad range of cosmetic oils. In general, any natural or synthetic oil for cosmetic use is suitable for the composition of present invention. Non-limiting examples of such as esters (e.g., alkyl benzoates having between12 to 15 carbons), triglycerides (e.g., Caprylic/Caprylate triglyceride), hydrocarbons (e.g., mineral oil, Petroleum Jelly), natural oils (e.g., Jojoba Oil, Safflower Oil, Olive Oil, Sweet Almond Oil, Mango Butter, Sunflower Oil, Aloe Butter, Cocoa Butter, Shea Butter, Palm Kernel Flakes, Beeswax, Lanolin Butter, Sweet Orange Oil, Lemon Oil, Avocado oil, Coconut Oil, Palm Oil, Sesame Oil, Peanut Oil, Peach Kernel Oil, Wheat Germ Oil, Macadamia Nut Oil, Night Primrose oil, Soya Oil and the derivatives thereof.), and castor oil, among others. Suitable oils are also disclosed, for example, at column 3, line 37, to column 4, line 4, of U.S. Patent No. 5,736,125; hereby incorporated by reference. Silicone oils may also be used as cosmetic oils.

Suitable synthetic oils are at least one member selected from the group consisting of fatty alcohols; fatty acid esters such as isopropyl myristate, palmitate, stearate and isostearate; oleyl oleate; isocetyl stearate; hexyl laurate; dibutyl adipate; dioctyl adiphate; myristyl myristate; oleyl erucate; polyethylene glycol and it derivatives; polyglyceryl fatty acid esters; and cetyl palmitate, by way of example only.

Silicone oils are also suitable. Useful silicone oils are non-volatile silicone oils known by INCI names that include dimethicone or dimethiconol. Volatile silicone oils such as cyclomethicones may also be used.

Water-in-oil emulsions can be prepared by mixing together (1) a heated (i.e., melted) solution of the oil phase and (2) an aqueous phase, the aqueous phase being at a temperature similar to the oil (typically within about 10 °C); and then cooling the mixture while stirring. While bringing the temperature of emulsion down Delivery System composition could be added below 45C as emulsion been emulsified and in process of adding other temperature sensitive cosmetic ingredients like preservative, actives. Alternatively, the Delivery System composition could be added to the aqueous phase if formulation is made without any heating like cold process formulations. Delivery System composition is generally added below 45 °C to room temperature in any cosmetic formulation. Regardless of the manner in which the Delivery System composition is added, the ratio of the aqueous phase to the oil phase can be, for example, about 0.5:1 to about 9:1.

The following are non-limiting examples of cosmetic formulations comprising water-in-oil emulsions:
A) Skin Moisturizer
   Water - about 50 to about 90 wt%
   Silicone - about 1 to about 10 wt%
   Emulsifier- about 0.5 to about 5 wt%
   Emollient - about 5 to about 20 wt%
   Delivery System composition - about 0.1 to about 20 wt%
   Polymer - about 0.1 to about 5 wt%
   Other Additives or Actives - about 0.1 to about 3 wt%
B) Sunscreen
   Water - about 50 to about 90 wt%
   Silicone - about 1 to about 10 wt%
   Emulsifier - about 0.5 to about 5 wt%
   Emollient - about 5 to about 20 wt%
   Delivery System composition - about 0.1 to about 20 wt%
   Polymer - about 0.1 to about 5 wt%
   Sunscreen Active - about 1 to about 25 wt%
   Other Additives or Actives - about 0.1 to about 3 wt%
C) Antiperspirant Deodorant
   Water - about 50 to about 90 wt%
   Silicone - up to about 10 wt%
   Emulsifier - about 0.5 to about 5 wt%
   Ethanol or other solvents - about 0 to about 20 wt%
   Delivery System composition - about 0.1 to about 20 wt%
   Polymer - up to about 5 wt%
   APDO actives - about 0.1 to about 30 wt%
   Other Additives or Actives - about 0.1 to about 5 wt%

Oil-in-water emulsions are prepared by mixing together (1) a heated (i.e., melted) solution of the oil phase and (2) an aqueous phase, the aqueous phase being at a temperature similar to the emollient solution (typically within about 10 °C); and then cooling the mixture while stirring. As with the water-in-oil emulsions, the Delivery System composition may initially be added to the aqueous phase if formulation is cold processable or added post-emulsification below 45 °C. The ratio of the oil phase to the water phase can be, for example, about 0.1:1 to about 1:1. The following are non-limiting examples of cosmetic formulations comprising oil-in-water emulsions:
A) Skin Moisturizer
   Water - about 50 to about 90 wt%
   Emulsifier- about 0.5 to about 5 wt%
   Emollient - about 1 to about 20 wt%
   Delivery System composition - about 0.1 to about 20 wt%
   Polymer - about 0.1 to about 5 wt%
   Other Additives or Actives - about 0.1 to about 3 wt%
B) Sunscreen
   Water - about 50 to about 90 wt%
   Emulsifier - about 0.5 to about 5 wt%
   Emollient - about 1 to about 20 wt%
   Ethanol and/or other organic solvent - up to about 35 wt%
   Delivery System composition - about 0.1 to about 20 wt%
   Polymer - about 0.1 to about 5 wt%
   Sunscreen Active - about 1 to about 25 wt%
   Other Additives or Actives - about 0.1 to about 3 wt%
C) Mousse or other hair styling product
   Water - about 50 to about 90 wt%
   Emulsifier - about 0.5 to about 1 wt%
   Surfactant - about 0.1 to about 2 wt%
   Delivery System composition - about 0.1 to about 20 wt%
   Polymer - up to about 5 wt%
   Other Additives or Actives - about 0.1 to about 2 wt%
   Solvent - about 1 to about 25 wt%
   Propellant - up to about 10 wt%
D) Antiperspirant Deodorant
   Water - about 50 to about 90 wt%
   Silicone - up to about 10 wt%
   Emulsifier - about 0.5 to about 5 wt%
   Ethanol or other solvents - about 1 to about 35 wt%
   Delivery System composition - about 0.1 to about 20 wt%
   Polymer - up to about 5 wt%
   APDO actives - about 0.1 to about 30 wt%
   Other Additives or Actives - about 0.1 to about 5 wt%

The following are non-limiting examples of cosmetic formulations comprising alcohol or aqueous systems.
A) Coloring Shampoo
   Water - about 50 to about 90 wt %
   Surfactant - about 2 to about 20 wt%
   Foam booster -up to about 20 wt%
   Delivery System composition - about 0.1 to about 20 wt%
   Polymer - up to about 5 wt%
   Other Additives or Actives - about 0.1 to about 10 wt%
B) Hair Spray (aerosol and non-aerosol)
   Water - about 10 to about 90 wt%
   Delivery System composition - about 0.1 to about 20 wt%
   Polymer - up to about 5 wt%
   Ethanol or other solvents - about 10 to about 90 wt%
   Optional Propellant for an aerosol - up to about 50 wt%
   Other Additives or Actives - about 0.1 to about 2 wt%
C) Shampoo
   Water - about 50 to about 90 wt%
   Surfactant - up to about 20 wt%
   Foam booster - about 2 to about 20 wt%
   Delivery System composition - about 0.1 to about 20 wt%
   Polymer - up to about 5 wt%
   Other Additives or Actives - about 0.1 to about 10 wt%
D) Hair Styling products
   Water - about 10 to about 90 wt%
   Delivery System composition - about 0.1 to about 20 wt%
   Polymer - about 0.1 to about 5 wt%
   Ethanol or other solvents- up to about 10 wt%
   Other Additives or Actives - about 0.1 to about 10 wt%
E) Body cleansing products
   Water - about 50 to about 90 wt%
   Surfactant - about 2 to about 20 wt%
   Delivery System composition - about 0.1 to about 20 wt%
   Polymer - up to about 5 wt%
   Foam booster - up to about 20 wt%
   Other Additives or Actives - about 0.1 to about 10 wt%.

In products in which emollients are employed, any suitable emollient for use in cosmetic compositions can be used. Examples of suitable emollients include at least one of esters (e.g., C12-15 alkyl benzoate) and triglycerides (e.g., Caprylic/caprylate triglyceride); hydrocarbon oils (e.g., mineral oil), natural oil (e.g., Jojoba oil, safflower oil), tridecyl trimellitate, sunflower oil, castor oil, among other compounds used to impart desired or improved sensory or aesthetic properties of a personal care composition. Many emollients used in cosmetic compositions also fall within the category of cosmetic oils.

In products in which emulsifiers are employed, any suitable cosmetic emulsifier having a hydrophilic-lipophilic balance (HLB) in the range of about 1 to about 20 can be used. The emulsifier can be nonionic, cationic, anionic, or amphoteric or a combination of such emulsifiers can be used.

Examples of nonionic emulsifiers are at least one of laureths, e.g. laureth-4; ceteths, e.g. ceteths-1; polyethylene glycol cetyl ether; Cetearth, e.g. cetearth-25; polyglycol fatty acid glycerides; hydroxylated lecithin; lactyl esters of fatty acids; and alkyl polyglycosides.

Examples of cationic emulsifiers are at least one of cetyldimethyl-2-hydroxyethylammonium dihydrogenphosphate; cetyltrimonium chloride; cetyltrimonium bromide; cocotrimonium methosulfate; as well as emulsifiers that contain quaternized nitrogen.

Anionic emulsifiers include, for example, at least one of alkyl sulfates; alkyl ether sulfates; alkylsulfonates; alkylarylsulfonates; alkyl succinates; alkyl sulfosuccinates; N-alkylsarcosinates; acyl taurates; acyl isethionates; alkyl phosphates; alkyl ether phosphates; alkyl ether carboxylates; alpha-olefinsulfonates, and the alkali metal and alkaline earth metal salts of such materials (e.g. sodium, potassium, magnesium, calcium) as well as ammonium, trialkylamine, trialkanol amine, and alkylalkanol amine salts. The alkyl ether sulfates, alkyl ether phosphates and alkyl ether carboxylates may include ethylene oxide or propylene oxide units.

Surfactants and/or foam boosters may also be employed and like the emulsifiers can be nonionic, cationic, anionic, or amphoteric or a combination of such surfactants can be used.

Suitable anionic surfactants are for example, at least one of alkyl sulfates; alkyl ether sulfates; alkylsulfonates; alkylarylsulfonates; alkyl succinates; N-alkylsarcosinates; acyl taurates; acyl isethionates; alkyl phosphates; alkyl ether phosphates; alkyl ether carboxylates; alpha olefinsulfonates, and may include the alkali metal and alkaline earth metal salts of such materials (e.g. sodium, potassium, magnesium, calcium) as well as ammonium, trialkylamine, trialkanol amine, and alkylalkanol amine salts. The alkyl ether sulfates, alkyl ether phosphates and alkyl ether carboxylates may include ethylene oxide or propylene oxide units.

Suitable amphoteric surfactants are, for example, at least one of alkylbetaines; alkylamidopropylbetaines; alkylsulfobetaines; alkyl glycinates; alkylcarboxyglycinates; alkyl amphoacetates or amphopropionates; and alkyl amphodiacetates or amphodipropionates. For example, it is possible to use cocodimethylsulfopropylbetaine, laurylbetaine, and cocamidopropylbetaine or sodium cocamphopropionate as surfactants.

Examples of nonionic surfactants are the reaction products of aliphatic alcohols having between 6 and 20 carbon atoms in the alkyl chains, which can be linear or branched with ethylene oxide and/or propylene oxide. Also suitable are at least one of alkylamine oxides; mono- or dialkylalkanolamides; fatty acid esters of polyethylene glycols; alkyl polyglycosides and sorbitan ether esters.

Examples of cationic surfactant are quaternary ammonium compounds, for example, cetyltrimethylammonium chloride, as well as other surfactants that contain a quaternized nitrogen.

In personal care compositions in which polymers are used with the Delivery System composition, any one or more polymer(s) can be of any type, including those which are at least one of nonionic, amphoteric or zwitterionic, anionic, cationic or mixtures of such types of polymers.

Exemplary synthetic polymers include at least one of vinyl pyrrolidone or acrylate homopolymer and copolymers, including carbomer, and those which have a vinyl acetate group or acrylate or acrylamide or taurate group. Natural non-ionic polymers suitable for the composition of the present invention can comprise at least one of cellulose, starches, chitosan, xanthan gum, guar gum, neutralized shellac and their derivatives. Cationic derivatives or chemical modified forms of natural polymers may also be included, such as chemically modifies starches, celluloses, guar and xanthan gum e.g., methylcellulose, hydroxyethylcellulose, quaternized guar.

The polymer may also include silicone compounds, such as, for example, at least one of polydiorganosiloxanes, polydialkylsiloxanes, polyalkylsiloxanes, polyarylsiloxane, silicone resins, silicone gums or dimethicone copolyols and amino-functional silicone compounds such as amodimethicone. Other silicone compounds include graft polymers of organosiloxane and polyethyloxazolines compounds known with the INCI name Polysilicone-9. Any polymeric compound having an INCI name including silicone, methicone, dimethicone, or siloxane as part of its name may be used.

In products in which propellant or solvents are employed, those may include at least one of isobutane, butane, dimethyl ether, and ethanol, among others.

Other Additives or Actives include one or more of additive compounds, active compounds, and moisturizing and humectants compounds. Examples of other additive compounds include one or more members selected from the group consisting of silicone based plasticizers, natural or synthetic compounds (e.g., polysaccharides, natural or synthetic gums, stabilizers, anionic and nonionic associative thickener or rheology modifiers soluble in oil or water phase), among other compounds. The additives may include at least one compound selected from the group consisting of preservatives, stabilizers (e.g., Xanthan Gum), antioxidant (e.g., Vitamins), rheology modifiers, fragrances, and pigments, among other additives.

In some personal care products, active compounds that interact with or protect skin or hair can be included. Examples of such active compounds include at least one of sunscreen compounds (e.g. zinc oxide, titanium dioxide, octinoxate, octocrylene, ethylhexyl salicylate, oxybenzone); skin whiteners (e.g. salicylic acid, aloesin, ethyl ascorbic acid, arbutin); anti-cellulite compounds; anti-aging compounds (e.g., polypeptides such as Argininie/Lysine, Argininie PCA, Aspergillus/Aspidosperma Quebracho Ferment, botanical actives, Bifida Ferment Lysate, Calophylum Inorhylum seed oil, camellia sinensis extract, ceramides, chlorella vulgaris extract, coriolus versicolor extract, corylus avellana (hazel) seed extract, erythorbic acid, hydrolyzed elastins, hydrolyzed proteins, hydrolyzed soy flour, hydrolyzed peptides, and Vitamins A, E, C, K, B7, and B5 as well as Niacinamide); anti-acne compounds (e.g., salicylic acid, sodium salicylate, benzoyl peroxide); anti-dandruff compounds (e.g., zinc pyrithione); APDO compounds (e.g., aluminum chlorohydrate, aluminum zirconium tetra chlorohydrex); vitamins (e.g., Tocopherol natural, synthetic Tocopherol, synthetic tocopheryl acetate, Retinol, Retinyl palmitate, Retinyl acetate, Provitamin B-5, ascorbic acid, sodium ascorbyl phosphate, Ascorbyl glucoside, Magnesium ascorbyl phosphate); insect repellents; and Coenzymes and Enzymes (e.g., Ubiquinone, Coenzyme Q10); Botanicals (e.g., Aloe vera, Green tea extract, Grape seed extract, Isoflavones, Chamomille/bisabolol, Fennel, Ginko, Ginseng, Guava); Alpha Hydroxy Acids (e.g., Citric acid, Glycolic acid, Lactic acid); Sugar cane extracts; Avena Sativa (Oat) Kernel Protein, and Avocado Sterols, proteins, peptides, copper peptides, fermented biopolymers, beta-glucan, caffeine, Hyaluronic acid and its derivatives like sodium hyaluronate, all by way of example only. It will be appreciated that certain active compounds may fall into more than one category and/or be used to accomplish more than one result.

Active compounds may also include at least one in vivo Fluorescent Dye. While an in vivo Fluorescent Dye may not interact with or protect skin or hair, it may be used as a model for cosmetic actives to allow for the assessment and prediction of epidermal penetration of cosmetic actives. In vivo Fluorescent Dyes include any fluorescent dye that meets regulatory and/or safety requirements for human in vivo skin penetration studies, and includes Fluorescein Alcon 10%, for example.

In personal care products, moisturizing and Humectants compounds that interact with or skin or hair could be simple or complex mixtures of chemical agents specially designed to make the external or upper layers of the skin (epidermis) softer and more pliable. They increase the skin's hydration or water content by reducing evaporation or by bringing moisture to the skin. Types of moisturizers and Humectants include, for example, naturally occurring skin lipids and sterols, as well as artificial or natural oils, emollients, lubricants. Examples of such moisturizing or humectants compounds include glycol (e.g., glycerin, propylene glycol, dipropylene glycol, butylene glycol MP diol, sorbitol, and hexylene glycol), Polysaccharides (e.g., Hyaluronic acid and its salts, B-1, 3-glucans, Chitosan), Botanicals (e.g., Aloe vera, Ginkgo, green tea extract, rose extract, sugar extract). Natural oils (e.g., coconut oil, grape seed oil, jojoba oil, olive oil, Argan oil), polyethylene glycol ether of methyl glucose (e.g., Methyl Gluceth-10), green algae, natural and herbal extract design for moisturization like alpha lipoic acid, peptides, proteins amino acids and collagens and betaine among others.

Personal care products may employ the Botanical Powder-Based Delivery System composition alone as a moisturizing effect and as a barrier affect on the skin good sensory attributes, and/or as another aspect of Botanical Powder-Based Delivery System in a cosmetic composition that further has a texturing agent in combination with other cosmetic ingredients. The Botanical Powder-Based Delivery System composition provides high hydration to skin by easily absorbing into the skin and enhancing a natural fresh look.

### Example 1. Partial Water Solubility of Lupine Powder

A botanical powder of Lupinus albus seed extract comprising 38 wt% proteins, 10 wt% polysaccharides, 14 wt% lipids and 28 wt% fibers was added at 2 wt% to deionized water and the mixture was heated at a rate of approximately 2 °C per minute to 80 ± 2 °C. The aqueous dispersion was maintained at 80 ± 2 °C and mixed at 350 to 450 rpm for 30 minutes, cooled to room temperature, and then was centrifuged for 30 minutes in an Eppendorf Centrifuge Model 5702 at 3000 rpm. An amount of ethanol equal in weight to the original weight of the centrifuged aqueous dispersion was added to the resultant precipitate, the sample was shaken by hand to wash the precipitate, and the sample was centrifuged for another 30 minutes. The precipitate recovered from the ethanol wash was dried to constant weight in a forced air oven at 50 °C. The ratio of dried weight of precipitate (recovered dry precipitate) to the initial weight of powder used to make the aqueous dispersion was 0.7, and thus the partial water solubility as determined at 80 °C was 0.3.

### Example 2. Preparation of a "Liposome 1" with Lupine Powder

| | |
|---|---|
| Deionized Water (g) | 140.4 |
| Glycerol (g) | 40.0 |
| Tetrasodium Glutamate Diacetate (g) | 0.2 |
| Phospholipid Type PL2 (g) | 13.4 |
| Phospholipid Type PL2, wt% of phosphatidylcholine | 25 |
| Lupinus Albus Seed Extract (g) | 4.0 |
| Preservative (g) | 2.0 |

The botanical powder of Example 1 was added to the water while stirring at 350 to 450 rpm and the mixture was heated at a rate of approximately 2 °C per minute to 80 ± 2 °C. The temperature and stirring were maintained for 30 minutes. Following this step the water and botanical powder mixture was cooled to room temperature. Tetrasodium Glutamate Diacetate and 97% of the indicated amount of Phospholipid type PL2 were added to the water and botanical powder mixture while homogenizing by means of an IKA T25 digital Ultra-Turrax at 14,000 rpm for 2 minutes. In a separate container, the remaining Phospholipid type PL2 was added to the glycerol and the mixture was heated to 78 ± 2 °C while stirring at 50 to 100 rpm. Stirring and temperature were maintained for 3-4 hours until the Phospholipid type PL2 was fully dissolved. Subsequently this mixture was cooled to room temperature. The solution of Phospholipid type PL2 and glycerol was slowly added to the aqueous phase while homogenizing at 14,000 rpm. Homogenization was continued for 3 minutes at ambient conditions. The preservative (Optiphen® BD from Ashland, Inc., comprised of 79 wt% benzyl alcohol, 12 wt% benzoic acid, 8 wt% dehydroactic acid) was added while homogenizing at 14,000 rpm. Homogenization was continued for an 2 additional minutes at ambient conditions. The particle size, as measured by Photon Correlation Spectroscopy as described previously, was 367 nm.

### Example 3. Reduced Sedimentation in Inventive "Liposome 1" Botanical Powder-Based Delivery System with Lupine Powder

An equal mass of the inventive composition of Example 2, and of the aqueous dispersion prepared in Example 1, both at room temperature, were centrifuged for 30 minutes in an Eppendorf Centrifuge Model 5702 at 3000 rpm. An amount of ethanol equal in weight to the original weight of the centrifuged Example 2 sample was added to the resultant precipitate, the sample was shaken by hand to wash the precipitate, and the sample was centrifuged for another 30 minutes. The precipitate recovered from the ethanol wash was dried to constant weight in a forced air oven at 50 °C. The composition of Example 2 exhibited a reduced sedimentation of 50% relative to Example 1.

### Example 4. Partial Water Solubility of Amaranth Flour

The procedure of Example 1 was repeated, except that Organic Amaranth Flour (obtained from Buffalo Valley Grains in Annandale, MN) was used in place of Lupinus albus seed extract. The ratio of dried weight of precipitate (recovered dry precipitate) to the initial weight of powder used to make the aqueous dispersion was 0.4, and thus the partial water solubility as determined at 80 °C was 0.6.

### Example 5. Preparation of a "Liposome 1" with Amaranth Flour

The composition and procedure of Example 2 was repeated, except that Organic Amaranth Flour (obtained from Buffalo Valley Grains in Annandale, MN) was used in place of Lupinus albus seed extract. The particle size, measured in accordance with Example 2, was 255 nm.

### Example 6. Reduced Sedimentation in Inventive "Liposome 1" Botanical Powder-Based Delivery System with Amaranth Flour

The procedure of Example 3 was repeated with the inventive composition of Example 5 in place of the inventive composition of Example 2, and the aqueous dispersion prepared in Example 4 in place of the aqueous dispersion prepared in Example 1. The composition of Example 5 exhibited a reduced sedimentation of 38% relative to Example 4.

### Example 7. Preparation of a "Cerasome" with Lupine Powder

| Ingredient | Amount |
|---|---|
| Deionized Water | 157.0 g |
| Ethanol | 33.0 g |
| Glycosphingolipids** | 6.0 g |
| Lupinus Albus Seed Extract | 4.0 g |

| | |
|---|---|
| ** vegetable Ceramides comprising 50 to 55 wt% glycosphingolipids | |

The botanical powder of Example 1 was added to the water while stirring at 350 to 450 rpm and the mixture was heated to 80 ± 2 °C. The temperature and stirring were maintained for 30 minutes. Following this step the water and botanical powder mixture was cooled to room temperature. In a separate container, Glycosphingolipids were added to the ethanol and the mixture was heated to 50 ± 2 °C while stirring at 50 to 100 rpm. Stirring and temperature were maintained for 15 min until the Glycosphingolipids were fully dissolved. The Glycosphingolipids solution was slowly added to the water and lupine mixture while homogenizing by means of an IKA T25 digital Ultra-Turrax at 14,000 rpm. Homogenization was continued for 5 minutes at ambient conditions. The particle size, measured in accordance with Example 2, was 234 nm.

### Example 8. Reduced Sedimentation in Inventive "Cerasome" Botanical Powder-Based Delivery System with Lupine Powder

The procedure of Example 3 is repeated with the inventive composition of Example 7 in place of the inventive composition of Example 2. The composition of Example 7 exhibits a reduced sedimentation relative to Example 1.

### Example 9. Preparation of a "Liposome II" with Lupine Powder

| | |
|---|---|
| Deionized Water (g) | 66.9 |
| 1,3-Butylene glycol (g) | 20.0 |
| Tetrasodium Glutamate Diacetate (g) | 0.1 |
| Phospholipid Type PL1 (g) | 10.0 |
| Phospholipid Type PL1, wt% of phosphatidylcholine | 92 |
| Lupinus Albus Seed Extract (g) | 2.0 |
| Preservative* (g) | 1.0 |

| | |
|---|---|
| * Preservative comprised 79 wt% benzyl alcohol, 12 wt% benzoic acid, 8 wt% dehydroacetic acid | |

The botanical powder of Example 1 was added to the water while stirring at 200 to 300 rpm and the mixture was heated to 80 ± 2 °C. The temperature and stirring were maintained for 30 minutes. Following this step the water and botanical powder mixture was cooled to room temperature and Tetrasodium Glutamate Diacetate was added to the water and botanical powder mixture while stirring at 200 to 300 rpm for 2 minutes. In a separate container, Phospholipid type PL1 was added to the 1,3-butylene glycol (Solvent) at ambient temperature and the mixture was occasionally stirred at 50 to 100 rpm for 45 minutes until the Phospholipid type PL1 was fully dissolved. The solution of Phospholipid type PL1 and Solvent was slowly added to the aqueous phase while homogenizing by means of an IKA T25 digital Ultra-Turrax at 14,000 rpm. Homogenization was continued for 3 minutes at ambient conditions. The preservative was added while homogenizing at 14,000 rpm, and homogenization was continued for 2 additional minutes at ambient conditions. The particle size, measured in accordance with Example 2, was 312 nm.

### Example 10. Reduced Sedimentation in Inventive "Liposome II" Botanical Powder-Based Delivery System with Lupine Powder

The procedure of Example 3 is repeated with the inventive composition of Example 9 in place of the inventive composition of Example 2. The composition of Example 9 exhibits a reduced sedimentation relative to Example 1.

### Example 11. Skin Cream Containing Inventive "Liposome I" Botanical Powder-Based Delivery System

| Ingredient | % weight |
|---|---|
| Composition of Example 2 | 3.00 |

| Phase A | |
|---|---|
| Deionized Water | balance |
| Glycerol | 5.00 |
| Xanthan Gum | 0.65 |

| Phase B | |
|---|---|
| Dicaprylyl Ether | 5.00 |
| Octyldodecanol | 3.00 |
| Isomerized Safflower Acid (Clarinol® A-80) | 3.00 |
| Arachidyl Alcohol, Behenyl Alcohol, Arachidyl Glucoside (Montanov™ 202) | 3.00 |

| Phase C | |
|---|---|
| Glyceryl Caprylate | 1.00 |

The ingredients of phase A were added together, mixed and heated to 75 °C. The ingredients of phase B were added to a separate container, heated to 75 °C until melted, and mixed. The phase B mixture was added to phase A while mixing, followed by homogenisation. The mixture was cooled to 60 °C and phase C was adding with gentle mixing. The mixture was further homogenised for a short time, cooled to 40 °C, and the Composition of Example 2 was added with gentle mixing until homogenous.

### Example 12. Second Skin Cream Containing Inventive Botanical Powder-Based Delivery System

A Skin Cream is prepared according to the composition and procedure of Example 11, except that the Composition of Example 2 is replaced by 5 wt% of any one of Example 5, Example 7, or Example 9.

### Example 13. Second Skin Cream Containing Inventive Botanical Powder-Based Delivery System

| Ingredient | % weight |
|---|---|
| Phase A | |
| Deionized Water | balance |
| Glycerol | 3.0 |

| Phase B | |
|---|---|
| Sodium Behenoyl Lactylate | 2.0 |
| Cetearyl Alcohol | 3.0 |
| Glyceryl Stearate | 2.6 |
| Isopropyl Palmitate | 6.0 |
| Simmondsia Chinensis Seed Oil | 6.0 |

| Phase C | |
|---|---|
| Composition of Example 2 | 10.0 |
| Preservative of Phenoxyethanol (71-77 wt%), Benzoic Acid (11-13 wt%), Dehydroxyacetic Acid (6.5-7.5 wt%), Ethylhexylglycerin (1.4-11.4 wt%), Polyaminopropyl Biguanide (0.1-1.1 wt%) [Euxyl® K702 from Schülke & Mayr GmbH] | 0.5 |

The ingredients of phase A were added together, mixed and heated to 80 °C. The ingredients of phase B were added to a separate container, mixed and heated to 80 °C. The phase B mixture was added to phase A while mixing and homogenizing. The mixture was cooled to 40 °C and ingredients of phase C were added with gentle mixing until the cream was homogeneous.

### Example 14. Third Skin Cream Containing Inventive Botanical Powder-Based Delivery System

A Skin Cream is prepared according to the composition and procedure of Example 13, except that the Composition of Example 2 is replaced by 5 wt% of any one of Example 5, Example 7, or Example 9.

### Example 15. Fourth Skin Cream Containing Inventive Botanical Powder-Based Delivery System

| Ingredient | % weight |
|---|---|
| Phase A | |
| Deionized Water | balance |
| Glycerol | 3.0 |
| Betaine | 1.5 |
| Xanthan Gum | 0.2 |

| Phase B | |
|---|---|
| Polyglyceryl-3 Methylglucose Distearate | 3.8 |
| Cetearyl Alcohol | 5.4 |
| Simmondsia Chinensis Seed Oil | 2.9 |
| Persea Gratissima Seed Oil | 4.2 |
| Mangifera Indica Seed Butter | 3.5 |

| Phase C | |
|---|---|
| Composition of Example 2 | 10.0 |
| Preservative of Phenoxyethanol (28.3-34.3 wt%), Benzyl Alcohol (28.3-34.3 wt%), Potassium Sorbate (17.0-20.6 wt%) [Euxyl® K700 from Schülke & Mayr GmbH] | 1.0 |

The Skin Cream was prepared according to the procedure of Example 13.

### Example 16. Fifth Skin Cream Containing Inventive Botanical Powder-Based Delivery System

A Skin Cream is prepared according to the composition and procedure of Example 15, except that the Composition of Example 2 is replaced by 5 wt% of any one of Example 5, Example 7, or Example 9.

### Example 17. Sixth Skin Cream Containing Inventive Botanical Powder-Based

Delivery System

| Ingredient | % weight |
|---|---|
| Phase A | |
| Deionized Water | balance |
| Triticum Vulgare Kernel Flour | 6.0 |

| Phase B | |
|---|---|
| Glyceryl Stearate Citrate | 4.3 |
| Cetearyl Alcohol | 3.6 |
| Decyl Cocoate | 3.0 |
| Simmondsia Chinensis Seed Oil | 5.2 |
| Dicaprylyl Carbonate | 2.3 |
| Dicaprylyl Ether | 1.7 |

| Phase C | |
|---|---|
| Composition of Example 2 | 10.0 |
| Preservative of Benzyl Alcohol (79 wt%), Benzoic Acid (12 wt%), Dehydroacetic (8 wt%) | 1.0 |

The Skin Cream was prepared according to the procedure of Example 13.

### Example 18. Seventh Skin Cream Containing Inventive Botanical Powder-Based Delivery System

A Skin Cream is prepared according to the composition and procedure of Example 17, except that the Composition of Example 2 is replaced by 5 wt% of any one of Example 5, Example 7, or Example 9.

### Example 19. Eighth Skin Cream Containing Inventive Botanical Powder-Based Delivery System

| Ingredient | % weight |
|---|---|
| Phase A | |
| Deionized Water | balance |
| Triticum Vulgare Kernel Flour | 6.0 |

| Phase B | |
|---|---|
| Brassica Campestris Seed Oil | 6.1 |
| Simmondsia Chinensis Seed Oil | 7.4 |
| Caprylic/Capric Triglyceride | 4.1 |
| Cetearyl Alcohol | 3.8 |
| Dicaprylyl Carbonate | 2.9 |
| Coco-Caprylate | 1.7 |

| Phase C | |
|---|---|
| Composition of Example 2 | 10.0 |
| Preservative of Benzyl Alcohol (79 wt%), Benzoic Acid (12 wt%), Dehydroacetic (8 wt%) | 1.0 |

The Skin Cream was prepared according to the procedure of Example 13.

### Example 20. Ninth Skin Cream Containing Inventive Botanical Powder-Based Delivery System

A Skin Cream is prepared according to the composition and procedure of Example 19, except that the Composition of Example 2 is replaced by 5 wt% of any one of Example 5, Example 7, or Example 9.

### Example 21. First Serum Containing Inventive Botanical Powder-Based Delivery System

| Ingredient | % weight |
|---|---|
| Phase A | |
| Deionized Water | balance |
| Carbomer | 1.3 |

| Phase B | |
|---|---|
| Sodium Hydroxide, 10 wt% in Water | 3.6 |

| Phase C | |
|---|---|
| Sorbeth-30 | 5.0 |
| Preservative of Phenoxyethanol (71-77 wt%), Benzoic Acid (11-13 wt%), Dehydroxyacetic Acid (6.5-7.5 wt%), Ethylhexylglycerin (1.4-11.4 wt%), Polyaminopropyl Biguanide (0.1-1.1 wt%) | 0.5 |
| Composition of Example 2 | 10.0 |

The ingredients of phase A were added together and mixed at room temperature until the Carbomer was fully dissolved. The ingredient of phase B was added to the mixture for neutralization. Ingredients of phase C were then added in the order given with gentle mixing until the serum was homogeneous.

### Example 22. Second Serum Containing Inventive Botanical Powder-Based Delivery System

A Serum is prepared according to the composition and procedure of Example 21, except that the Composition of Example 2 is replaced by 5 wt% of any one of Example 5, Example 7, or Example 9.

### Example 23. Tenth Skin Cream Containing Inventive Botanical Powder-Based

Delivery System

| Ingredient | % weight |
|---|---|
| Phase A | |
| Deionized Water | balance |
| Xanthan Gum | 0.9 |

| Phase B | |
|---|---|
| Isopropyl Myristate | 10.0 |
| Jojoba Oil | 1.0 |
| Cyclopentasiloxane | 5.0 |
| Cetearyl Alcohol (and) Dicetyl Phosphate (and) Ceteth-10 Phosphate | 6.0 |
| Bis-PEG/PPG-14/14 Dimethicone | 2.5 |
| Apricot Kernel Oil | 0.4 |
| Sweet Almond Oil | 0.6 |

| Phase C | |
|---|---|
| Composition of Example 2 | 3.0 |
| Propylene glycol, Diazolidinyl urea, Methylparaben, Propylparaben | 1.0 |
| Triethanolamine | as needed to achieve final pH 5.7 |

The ingredients of phase A were added together, mixed and heated to 85 °C. The ingredients of phase B were added to a separate container, mixed and heated to 85 °C. The phase B mixture was added to phase A and mixed for 3 minutes, cooled to 65 °C, and homogenized at 6000 rpm for 3 minutes. The mixture was cooled with stirring to between 40 °C and 20 °C, and ingredients of phase C were then added in the order given with gentle mixing until the cream was homogeneous.

### Example 24. Eleventh Skin Cream Containing Inventive Botanical Powder-Based Delivery System

A Skin Cream is prepared according to the composition and procedure of Example 23, except that the Composition of Example 2 is replaced by 5 wt% of any one of Example 5, Example 7, or Example 9.

### Example 25. Twelfth Skin Cream Containing Inventive Botanical Powder-Based Delivery System

| Ingredient | % weight |
|---|---|
| Phase A | |
| Deionized Water | balance |
| Xanthan Gum | 0.9 |

| Phase B | |
|---|---|
| Mineral Oil | 12.5 |
| PEG-30 dipolyhydroxy stearate | 1.0 |
| Steareth-2 | 2.0 |
| Steareth 21 | 1.0 |
| Dimethicone | 4.5 |
| C12-15 Alkyl Benzoate | 4.5 |

| Phase C | |
|---|---|
| Composition of Example 2 | 3.0 |
| DMDM Hydantoin | 0.5 |

The Skin Cream was prepared according to the procedure of Example 23.

### Example 26. Thirteenth Skin Cream Containing Inventive Botanical Powder-Based Delivery System

A Skin Cream is prepared according to the composition and procedure of Example 25, except that the Composition of Example 2 is replaced by 5 wt% of any one of Example 5, Example 7, or Example 9.

### Example 27. Fourteenth Skin Cream Containing Inventive Botanical Powder-Based Delivery System

| Ingredient | % weight |
|---|---|
| Phase A | |
| Deionized Water | balance |
| Xanthan Gum | 0.2 |

| Phase B | |
|---|---|
| Octinoxate | 1.0 |
| Cetearyl Alcohol | 6.0 |
| Ceteareth-25 | 0.5 |

| Phase C | |
|---|---|
| Composition of Example 2 | 3.0 |
| Propylene Glycol, Diazolidinyl Urea, Methyparaben, Propylparaben | 1.0 |

The Skin Cream was prepared according to the procedure of Example 23.

### Example 28. Fifteenth Skin Cream Containing Inventive Botanical Powder-Based Delivery System

A Skin Cream is prepared according to the composition and procedure of Example 27, except that the Composition of Example 2 is replaced by 5 wt% of any one of Example 5, Example 7, or Example 9.

### Example 29. Sunscreen (SPF 15) Containing Inventive Botanical Powder-Based Delivery System

| Ingredient | % weight |
|---|---|
| Phase A | |
| Deionized Water | balance |
| Acrylates/C10-30 Alkyl Acrylate Crosspolymer | 0.3 |
| Acrylate polymer | 1.0 |
| Triethanolamine | 1.0 |

| Phase B | |
|---|---|
| Octocrylene | 3.0 |
| Octisalate | 3.0 |
| Oxybenzone | 2.0 |
| Avobenzone | 1.0 |
| Stearic Acid | 2.0 |
| Glyceryl Monostearate SE | 3.0 |
| Dimethicone | 0.5 |
| C12-15 Alkyl Benzoate | 8.0 |

| Phase C | |
|---|---|
| Composition of any one of Example 2, Example 5, Example 7, or Example 9 | 5.0 |
| Phenoxyethanol & Ethylhexylglycerin | 1.0 |

The Sunscreen is prepared according to the procedure of Example 23.

### Example 30. Hair Styling and Moisturizing Cream Containing Inventive Botanical Powder-Based Delivery System

| Ingredient | % weight |
|---|---|
| Phase A | |
| Deionized Water | balance |
| Carbomer | 0.5 |
| Triethanolamine | 0.8 |
| Polyurethane-2 and Polymethyl Methacrylate | 5.0 |
| Polyquaternium-55 | 1.0 |

| Phase B | |
|---|---|
| Ceteareth-20 | 0.5 |
| Polysilicone-11 & Water & Laureth-12 & Phenoxyethanol & Ethylhexylglycerin | 4.0 |
| Dimethicone 100 Cst | 0.1 |
| PEG-12 Dimethicone | 5.0 |
| Jojoba oil | 2.0 |

| Phase C | |
|---|---|
| Composition of any one of Example 2, Example 5, Example 7, or Example 9 | 5.0 |
| Phenoxyethanol & Ethylhexylglycerin | 1.0 |

The Hair Cream is prepared according to the procedure of Example 23.

### Example 31. Moisturizing BB Cream (Foundation) Containing Inventive Botanical Powder-Based Delivery System

| Ingredient | % weight |
|---|---|
| Phase A | |
| Polysilicone-11 & Water & Laureth-12 & Phenoxyethanol & Ethylhexylglycerin | 5.0 |
| Dimethicone 50 Cst | 20.0 |
| PEG-10 Dimethicone | 2.0 |
| Dimethicone & PEG/PPG-18/18 Dimethicone | 2.0 |
| Disteardimonium Hectorite | 0.6 |

| Phase B | |
|---|---|
| Yellow Iron Oxide | 1.3 |
| Red Iron Oxide | 0.4 |
| Black Iron Oxide | 0.1 |
| Titanium Dioxide & Methicone | 6.6 |
| Mica | 6.6 |
| Dimethicone 50 Cst | 7.5 |
| PEG-10 Dimethicone | 2.0 |

| Phase C | |
|---|---|
| Deionized Water | balance |
| Glycerin | 5.0 |
| Butylene Glycol | 3.0 |
| Sodium Chloride | 1.0 |

| Phase D | |
|---|---|
| Composition of any one of Example 2, Example 5, Example 7, or Example 9 | 3.0 |
| Phenoxyethanol & Ethylhexylglycerin | 1.0 |

The ingredients of phase A are added together, are mixed and heated to 85 °C. The ingredients of phase B are added to a separate container, are mixed and heated to 85 °C. The phase B mixture is added to phase A and is mixed for 3 minutes; the mixture is cooled to 65 °C, and is homogenized at 6000 rpm for 3 minutes. Phase C ingredients are then added to the mixture and mixing is continued until homogeneous. The mixture is cooled with stirring to between 40 °C and 20 °C, and ingredients of phase D are then added with gentle mixing until the cream is homogeneous.

### Example 32. Hair Conditioner with Moisturization Containing Inventive Botanical Powder-Based Delivery System

| Ingredient | % weight |
|---|---|
| Phase A | |
| Deionized Water | balance |
| Polyquaternium-10 | 0.5 |
| Polyquaternium-4 | 0.7 |

| Phase B | |
|---|---|
| PEG 12 Dimethicone | 2.0 |
| Cyclopentasiloxane (and) Cetearyl Dimethicone/Vinyl Dimethicone Crosspolymer | 4.5 |
| Composition of any one of Example 2, Example 5, Example 7, or Example 9 | 3.0 |
| Phenoxyethanol & Ethylhexylglycerin | 1.0 |

The ingredients of phase A are added together and mixed until homogeneous. The ingredient of phase B are added together in a separate container and mixed until homogeneous. Phase B is added to phase A and mixed until the conditioner is homogeneous.

### Example 33. Partial Water Solubility of Other Botanical Powders

The procedure of Example 1 is repeated, except that Lupinus albus seed extract is replaced by any one of the following Botanical Powders: alfalfa (genus Medicago), amaranth (genus Amaranthus), argan (genus Argania), arrowroot, barley (genus Hordeum), lupine (genus Lupinus, other than Lupinus albus), oat (genus Avena), rice (genus Oryza), soya (genus Glycine), pea (genus Pisum), and wheat (genus Triticum). The ratio of dried weight of precipitate (recovered dry precipitate) to the initial weight of powder in the aqueous dispersion is measured and the partial water solubility at 80 °C is determined.

### Example 34. Preparation of a "Liposome 1" with Other Botanical Powders

The composition and procedure of Example 2 is repeated, except that Lupinus albus seed extract is replaced by the Botanical Powder of Example 33.

### Example 35. Reduced Sedimentation in Inventive "Liposome 1" Botanical Powder-Based Delivery System with Other Botanical Powders

The procedure of Example 3 is repeated with the inventive composition of Example 34 in place of the inventive composition of Example 2, and the aqueous dispersion of Example 33 in place of the aqueous dispersion prepared in Example 1. The composition of Example 34 exhibits a reduced sedimentation relative to Example 33.

### Example 36. Preparation of a "Cerasome" with Other Botanical Powders

The composition and procedure of Example 7 is repeated, except that Lupinus albus seed extract is replaced by the Botanical Powder of Example 33.

### Example 37. Reduced Sedimentation in Inventive "Cerasome" Botanical Powder-Based Delivery System with Lupine Powder

The procedure of Example 3 is repeated with the inventive composition of Example 36 in place of the inventive composition of Example 2, and the aqueous dispersion of Example 33 in place of the aqueous dispersion prepared in Example 1. The composition of Example 36 exhibits a reduced sedimentation relative to Example 33.

### Example 38. Preparation of a "Liposome II" with Other Botanical Powders

The composition and procedure of Example 9 is repeated, except that Lupinus albus seed extract is replaced by the Botanical Powder of Example 33.

### Example 39. Reduced Sedimentation in Inventive "Liposome 1" Botanical Powder-Based Delivery System with Other Botanical Powders

The procedure of Example 3 is repeated with the inventive composition of Example 38 in place of the inventive composition of Example 2, and the aqueous dispersion of Example 33 in place of the aqueous dispersion prepared in Example 1. The composition of Example 38 exhibits a reduced sedimentation relative to Example 33.

### Examples 40 - 45. Preparations of Glycerol-Based Liposomal Delivery System

Preparations of Examples 40 - 45 were prepared as follows. In a first process step, the glycerol was mixed with water while stirring at 200 to 300 rpm and the water/glycerol mixture was heated to 80 ± 5 °C. The Phospholipids were slowly added at a temperature 80 ± 5 °C while homogenizing by means of an IKA T25 digital Ultra-Turrax at 14,000 rpm. Homogenization was continued for 4 minutes. Subsequently this mixture was cooled to ambient temperature and the preservative (79 wt% benzyl alcohol, 12 wt% benzoic acid, 8 wt% dehydroactic acid, Optiphen® BD from Ashland, Inc.) was added while stirring at 200 to 300 rpm. Then the pH value of the mixture was adjusted, with continued stirring at 200 to 300 rpm, with 30% caustic soda solution dropwise to a pH of 5.5 ± 0.3 °C. Finally, the mixture was homogenized by means of a Niro Soavi Panda 2K high pressure homogenizer for three cycles of 2 minutes each at a pressure of 400 bar. The particle size was measured by Photon Correlation Spectroscopy as described previously.

| | Example 40 | Example 41 | Example 42 | Example 43 | Example 44 | Example 45 |
|---|---|---|---|---|---|---|
| Deionized Water (g) | 296 | 196 | 288 | 294 | 288 | 296 |
| Glycerol (g) | 80 | 160 | 100 | 80 | 100 | 80 |
| Phospholipid Type PL1 (g) | 20 | 40 | 8.0 | 22 | 8.0 | 20 |
| Phospholipid Type PL1, wt% of phosphatidylcholine | 92 | 92 | 92 | 92 | 94 | 94 |
| Preservative* (g) | 4.0 | 4.0 | 4.0 | 4.0 | 4.0 | 4.0 |
| Particle size, nm | 120 | 113 | 110 | 128 | 126 | 134 |

| | | | | | | |
|---|---|---|---|---|---|---|
| * Preservative comprised 79 wt% benzyl alcohol, 12 wt% benzoic acid, 8 wt% dehydroacetic acid | | | | | | |

### Examples 46 - 51. Preparations of Glycerol-Based Liposomal Delivery System Containing an Active Compound

Preparations of Examples 46-51 are prepared as follows. In a first process step, the glycerol is mixed with water while stirring at 200 to 300 rpm and the water/glycerol mixture is heated to 80 ± 5 °C. The Fluorescein Alcon is added to the water/glycerol mixture while stirring at 200 to 300 rpm, and the Phospholipids are slowly added at a temperature 80 ± 5 °C while homogenizing by means of an IKA T25 digital Ultra-Turrax at 14,000 rpm. Homogenization is continued for 4 minutes. Subsequently this mixture is cooled to ambient temperature and the preservative (79 wt% benzyl alcohol, 12 wt% benzoic acid, 8 wt% dehydroactic acid, Optiphen® BD from Ashland, Inc.) is added while stirring at 200 to 300 rpm. Then the pH value of the mixture is adjusted, while stirring at 200 to 300 rpm, with 30% caustic soda solution dropwise to a pH of 5.5 ± 0.3 °C. Finally, the mixture is homogenized by means of a Niro Soavi Panda 2K high pressure homogenizer for three cycles of 2 minutes each at a pressure of 400 bar. The particle size is measured by Photon Correlation Spectroscopy as described previously.

Targeted topical epidermal delivery of each example composition is measured by 5D-IVT as described previously, and the amount of Fluorescin Alcon present in 5D-IVT images at deeper skin layers is increased relative to a control (an equivalent amount of Fluorescin Alcon applied to the skin from an aqueous solution).

| | Example 46 | Example 47 | Example 48 | Example 49 | Example 50 | Example 51 |
|---|---|---|---|---|---|---|
| Deionized Water (g) | 296 | 196 | 288 | 294 | 288 | 296 |
| Glycerol (g) | 80 | 160 | 100 | 80 | 100 | 80 |
| Fluorescein Alcon (g) | 0.0016 | 0.0016 | 0.0016 | 0.0016 | 0.0016 | 0.0016 |
| Phospholipid Type PL1 (g) | 20 | 40 | 8.0 | 22 | 8.0 | 20 |
| Phospholipid Type PL1, wt% of phosphatidylcholine | 92 | 92 | 92 | 92 | 94 | 94 |
| Preservative* (g) | 4.0 | 4.0 | 4.0 | 4.0 | 4.0 | 4.0 |

| | | | | | | |
|---|---|---|---|---|---|---|
| * Preservative comprised 79 wt% benzyl alcohol, 12 wt% benzoic acid, 8 wt% dehydroacetic acid Example 52. Preparation of Glycerol-Ethanol-Based Liposomal Delivery System | | | | | | |

Example 52 was prepared as follows. In a first process step, the glycerol was mixed with water while stirring at 200-300 rpm at ambient temperature; the Fluorescein Alcon was then added to the water/glycerol mixture while stirring at 200 to 300 rpm. In a separate container, the Phospholipid was added to the ethanol at ambient temperature and the mixture was stirred occassionally at 50 to 100 rpm over a period of 45 minutes until the Phospholipid was fully dissolved. The ethanolic solution of Phospholipid was slowly added to the aqueous phase while homogenizing by means of an IKA T25 digital Ultra-Turrax at 14,000 rpm. Homogenization was continued for 4 minutes. Subsequently the preservative (79 wt% benzyl alcohol, 12 wt% benzoic acid, 8 wt% dehydroactic acid, Optiphen® BD from Ashland, Inc.) was added while stirring at 200 to 300 rpm for 2 minutes. The Potassium Dihydrogen Phosphate and Sodium Hydroxide solution were added to 3 grams of water in a separate container, and stirred until fully dissolved to produce a premix, the premix then slowly added to the homogenized batch and stirred at 200 to 300 rpm for 3 minutes. The particle size was measured by Photon Correlation Spectroscopy as described previously.

Targeted topical epidermal delivery of the composition was measured by 5D-IVT as described previously, and the amount of Fluorescin Alcon present in 5D-IVT images at deeper skin layers was increased relative to a control (an equivalent amount of Fluorescin Alcon applied to the skin from an aqueous solution).

| | Example 52 |
|---|---|
| Deionized Water (g) | 65.07 |
| Ethanol (g) | 3.33 |
| Glycerol (g) | 20.0 |
| Fluorescein Alcon (g) | 0.0004 |
| Potassium Dihydrogen Phosphate (g) | 0.5 |
| Sodium Hydroxide, 30% in Water (g) | 0.1 |
| Phospholipid Type PL1 (g) | 10.0 |
| Phospholipid Type PL1, wt% of phosphatidylcholine | 76.5 |
| Preservative* (g) | 1.0 |
| Particle size, nm | 146 |

| | |
|---|---|
| * Preservative comprised 79 wt% benzyl alcohol, 12 wt% benzoic acid, 8 wt% dehydroacetic acid | |

### Examples 53 - 54. Preparations of Glycol-Based Liposomal Delivery System

Preparations of Examples 53 - 54 were prepared as follows. In a first process step, the Fluorescein Alcon was added to the water phase while stirring at 200 to 300 rpm. In a separate container, the Phospholipid was added to the glycol at ambient temperature and the mixture was stirred occasionally at 50 to 100 rpm over a period of 45 minutes until the Phospholipid was fully dissolved. The glycolic solution of Phospholipid was slowly added to the aqueous phase while homogenizing by means of an IKA T25 digital Ultra-Turrax at 14,000 rpm. Homogenization was continued for 4 minutes. Subsequently the preservative (79 wt% benzyl alcohol, 12 wt% benzoic acid, 8 wt% dehydroactic acid, Optiphen® BD from Ashland, Inc.) was added while stirring at 200 to 300 rpm for 2 minutes. Potassium Dihydrogen Phosphate and Sodium Hydroxide solution was added to 3 grams of water in a separate container, and stirred until fully dissolved to produce a premix, the premix then slowly added to the homogenized batch and stirred at 200 to 300 rpm for 3 minutes. The particle size was measured by Photon Correlation Spectroscopy as described previously.

| | Example 53 | Example 54 |
|---|---|---|
| Deionized Water (g) | 68.4 | 68.4 |
| 1,3-Butylene glycol (g) | | 20.0 |
| 1,2-Pentylene glycol (g) | 20.0 | |
| Fluorescein Alcon (g) | 0.0004 | 0.0004 |
| Potassium Dihydrogen Phosphate (g) | 0.5 | 0.5 |
| Sodium Hydroxide, 30% in Water (g) | 0.1 | 0.1 |
| Phospholipid Type PL1 (g) | 10.0 | 10.0 |
| Phospholipid Type PL1, wt% of phosphatidylcholine | 92 | 92 |
| Preservative* (g) | 1.0 | 1.0 |
| Particle size, nm | 309 | 132 |

| | | |
|---|---|---|
| * Preservative comprised 79 wt% benzyl alcohol, 12 wt% benzoic acid, 8 wt% dehydroacetic acid | | |

### Example 55. Measurement of Targeted Epidermal Delivery of Example 53

Targeted topical epidermal delivery of the composition of Example 53 was measured by 5D-IVT as described previously, and the amount of Fluorescin Alcon present in 5D-IVT images at deeper skin layers was increased relative to a control (an equivalent amount of Fluorescin Alcon applied to the skin from an aqueous solution).

### Example 56. Measurement of Targeted Epidermal Delivery of Example 54

Targeted topical epidermal delivery of the composition of Example 54 is measured by 5D-IVT as described previously, and the amount of Fluorescin Alcon present in 5D-IVT images at deeper skin layers is increased relative to a control (an equivalent amount of Fluorescin Alcon applied to the skin from an aqueous solution).

### Example 57. Preparation of Glycerol-Based Liposomal Delivery System

Example 57 was prepared as follows. The Phospholipid Type PL1 was added to the glycerol and the mixture was heated at a rate of approximately 2 °C per minute to 80 ± 5 °C while stirring at 100 to 150 rpm. At 80 ± 5 °C ubiquinone was added while stirring at 200 to 300 rpm, and the mixture was subsequently homogenized by means of an IKA T25 digital Ultra-Turrax at 14,000 rpm for 2 minutes. The deionized water was slowly added to the mixture containing glycerol, ubiquinone and Phospholipid Type PL1 at a temperature 80 ± 5 °C while homogenizing by means of an IKA T25 digital Ultra-Turrax at 14,000 rpm. Homogenization was continued for 4 minutes. Subsequently this mixture was cooled to ambient temperature and the preservative (79 wt% benzyl alcohol, 12 wt% benzoic acid, 8 wt% dehydroactic acid, Optiphen® BD from Ashland, Inc.) was added while stirring at 200 to 300 rpm. Then the pH value of the mixture was adjusted, with continued stirring at 200 to 300 rpm, with 30% caustic soda solution dropwise to a pH of 5.5 ± 0.3 °C. Finally, the mixture was homogenized by means of a Niro Soavi Panda 2K high pressure homogenizer for three cycles of 2 minutes each at a pressure of 400 bar. The particle size was measured by Photon Correlation Spectroscopy as described previously.

### Examples 58 - 61. Preparations of Glycerol-Based Liposomal Delivery System

Examples 58 - 61 are prepared following the procedure of Example 57. The particle size is measured by Photon Correlation Spectroscopy as described previously.

| | Example 57 | Example 58 | Example 59 | Example 60 | Example 61 |
|---|---|---|---|---|---|
| Deionized Water (g) | 242.5 | 292.5 | 267.5 | 217.5 | 192.5 |
| Glycerol (g) | 200 | 150 | 175 | 225 | 250 |
| Ubiquinone (g) | 2.5 | 2.5 | 2.5 | 2.5 | 2.5 |
| Phospholipid Type PL1 (g) | 50 | 50 | 50 | 50 | 50 |
| Phospholipid Type PL1, wt% of phosphatidylcholine | 92 | 92 | 92 | 92 | 92 |
| Preservative* (g) | 5.0 | 5.0 | 5.0 | 5.0 | 5.0 |
| Particle size, nm | 102 | 109 | 114 | 88 | 80 |

| | | | | | |
|---|---|---|---|---|---|
| * Preservative comprised 79 wt% benzyl alcohol, 12 wt% benzoic acid, 8 wt% dehydroacetic acid | | | | | |

### Example 62. Partial Water Solubility of Additional Botanical Powders

The procedure of Example 1 is repeated, except that Lupinus albus seed extract is replaced by any one of the following Botanical Powders: baobab (genus Adansonia), hop (genus Humulus), maize (genus Zea), chick pea (genus Cicer), apple (genus Pyrus), citrus (genus citrus), coconut (genus Cocos). The ratio of dried weight of precipitate (recovered dry precipitate) to the initial weight of powder in the aqueous dispersion is measured and the partial water solubility at 80 °C is determined.

### Examples 63 - 70. Preparations of a "Liposome 1" with Additional Botanical Powders

Preparations of Examples 63 - 70 were prepared following the procedure of Example 2. The particle size was measured by Photon Correlation Spectroscopy as described previously.

| | Example 63 | Example 64 | Example 65 | Example 66 |
|---|---|---|---|---|
| Deionized Water (g) | 70.2 | 70.2 | 70.2 | 70.2 |
| Glycerol (g) | 20.0 | 20.0 | 20.0 | 20.0 |
| Tetrasodium Glutamate Diacetate (g) | 0.1 | 0.1 | 0.1 | 0.1 |
| Phospholipid Type PL2 (g) | 6.7 | 6.7 | 6.7 | 6.7 |
| Phospholipid Type PL2, wt% of phosphatidylcholine | 25 | 25 | 25 | 25 |
| Cococin (Coconut water solids) | 2.0 | - | - | - |
| Maize Flour | - | 2.0 | - | - |
| Chick Pea Flour | - | - | 2.0 | - |
| Pea Protein | - | - | - | 2.0 |
| Preservative* (g) | 1.0 | 1.0 | 1.0 | 1.0 |
| Particle size, nm | 276 | 209 | 315 | 399 |

| | | | | |
|---|---|---|---|---|
| * Preservative comprised 79 wt% benzyl alcohol, 12 wt% benzoic acid, 8 wt% dehydroacetic acid | | | | |

| | Example 67 | Example 68 | Example 69 | Example 70 |
|---|---|---|---|---|
| Deionized Water (g) | 70.2 | 70.2 | 70.2 | 70.2 |
| Glycerol (g) | 20.0 | 20.0 | 20.0 | 20.0 |
| Tetrasodium Glutamate Diacetate (g) | 0.1 | 0.1 | 0.1 | 0.1 |
| Phospholipid Type PL2 (g) | 6.7 | 6.7 | 6.7 | 6.7 |
| Phospholipid Type PL2, wt% of phosphatidylcholine | 25 | 25 | 25 | 25 |
| Soy Sprout Extract | 2.0 | - | - | - |
| Hop Flower Extract | - | 2.0 | - | - |
| Apple Fiber | - | - | 2.0 | - |
| Citrus Fiber | - | - | - | 2.0 |
| Preservative* (g) | 1.0 | 1.0 | 1.0 | 1.0 |
| Particle size, nm | 379 | 190 | 222 | 244 |

| | | | | |
|---|---|---|---|---|
| * Preservative comprised 79 wt% benzyl alcohol, 12 wt% benzoic acid, 8 wt% dehydroacetic acid | | | | |

### Examples 71 - 77. Preparations of a "Cerasome" with Additional Botanical Powders

Preparations of Examples 71 - 77 were prepared following the procedure of Example 7. The particle size was measured by Photon Correlation Spectroscopy as described previously.

| | Example 71 | Example 72 | Example 73 | Example 74 |
|---|---|---|---|---|
| Deionized Water (g) | 78.5 | 78.5 | 78.5 | 78.5 |
| Ethanol (g) | 16.5 | 16.5 | 16.5 | 16.5 |
| Glycosphingolipids** (g) | 3.0 | 3.0 | 3.0 | 3.0 |
| Baobab Fruit Extract | 2.0 | - | - | - |
| Cococin (Coconut water solids) | - | 2.0 | - | - |
| Chick Pea Flour | - | - | 2.0 | - |
| Pea Protein | - | - | - | 2.0 |
| Particle size, nm | 224 | 252 | 223 | 243 |

| | | | | |
|---|---|---|---|---|
| ** vegetable Ceramides comprising 50 to 55 wt% glycosphingolipids | | | | |

| | Example 75 | Example 76 | Example 77 |
|---|---|---|---|
| Deionized Water (g) | 78.5 | 78.5 | 78.5 |
| Ethanol (g) | 16.5 | 16.5 | 16.5 |
| Glycosphingolipids** (g) | 3.0 | 3.0 | 3.0 |
| Soy Sprout Extract | 2.0 | - | - |
| Hop Flower Extract | - | 2.0 | - |
| Citrus Fiber | - | - | 2.0 |
| Particle size, nm | 233 | 193 | 254 |

| | | | |
|---|---|---|---|
| ** vegetable Ceramides comprising 50 to 55 wt% glycosphingolipids | | | |

### Examples 78 - 85. Preparations of a "Liposome 2" with Additional Botanical Powders

Preparations of Examples 78 - 85 were prepared as follows. The Botanical Powder (one of Baobab Fruit Extract, Cococin, Maize Flour, Pea Protein, Hop Flower Extract, Soy Sprout Extract, Apple Fiber, Citrus Fiber) was added to the water while stirring at 200 to 300 rpm and the mixture was heated at a rate of approximately 2 °C per minute to 80 ± 2 °C. The temperature and stirring were maintained for 30 minutes. Following this step the water and extract mixture was cooled to room temperature. In a separate container, Phospholipid type PL1 was added to the ethanol at ambient temperature and the mixture was occasionally stirred at 50 to 100 rpm for 45 minutes until the Phospholipid type PL1 was fully dissolved. The solution of Phospholipid type PL1 and ethanol was slowly added to the aqueous phase while homogenizing by means of an IKA T25 digital Ultra-Turrax at 14,000 rpm. Homogenization was continued for 3 minutes at ambient conditions. Potassium Dihydrogen Phosphate and Sodium Hydroxide solution was added to 3 grams of water in a separate container, and stirred until fully dissolved to produce a premix, the premix then slowly added to the homogenized batch and stirred at 200 to 300 rpm for 5 minutes. The particle size was measured by Photon Correlation Spectroscopy as described previously.

| | Example 78 | Example 79 | Example 80 | Example 81 |
|---|---|---|---|---|
| Deionized Water (g) | 70.85 | 70.85 | 70.85 | 70.85 |
| Ethanol (g) | 16.5 | 16.5 | 16.5 | 16.5 |
| Phospholipid Type PL1 (g) | 10.0 | 10.0 | 10.0 | 10.0 |
| Phospholipid Type PL1, wt% of phosphatidylcholine | 78 | 78 | 78 | 78 |
| Potassium Dihydrogen Phosphate (g) | 0.5 | 0.5 | 0.5 | 0.5 |
| Sodium Hydroxide, 30% in Water (g) | 0.15 | 0.15 | 0.15 | 0.15 |
| Baobab Fruit Extract | 2.0 | - | - | - |
| Cococin (Coconut water solids) | - | 2.0 | - | - |
| Maize Flour | - | - | 2.0 | - |
| Pea Protein | - | - | - | 2.0 |
| Particle size, nm | 239 | 242 | 190 | 218 |

| | Example 82 | Example 83 | Example 84 | Example 85 |
|---|---|---|---|---|
| Deionized Water (g) | 70.85 | 70.85 | 70.85 | 70.85 |
| Ethanol (g) | 16.5 | 16.5 | 16.5 | 16.5 |
| Phospholipid Type PL1 (g) | 10.0 | 10.0 | 10.0 | 10.0 |
| Phospholipid Type PL1, wt% of phosphatidylcholine | 78 | 78 | 78 | 78 |
| Potassium Dihydrogen Phosphate (g) | 0.5 | 0.5 | 0.5 | 0.5 |
| Sodium Hydroxide, 30% in Water (g) | 0.15 | 0.15 | 0.15 | 0.15 |
| Soy Sprout Extract | 2.0 | - | - | - |
| HopFlower Extract | - | 2.0 | - | - |
| Apple Fiber | - | - | 2.0 | - |
| Citrus Fiber | - | - | - | 2.0 |
| Particle size, nm | 219 | 220 | 215 | 209 |

While the invention has been described with reference to certain embodiments, it will be understood by those skilled in the art that various changes may be made and equivalents may be substituted for elements thereof without departing from the scope of the invention. In addition, many modifications may be made to adapt a particular situation or material to the teachings of the invention without departing from the essential scope thereof. Therefore, it is intended that the invention not be limited to the particular embodiment disclosed as a preferred mode contemplated for carrying out this invention, but that the invention will include all embodiments to be used alone as well in combinations with each other and falling within the scope of the appended claims.

The invention also relates to the following numbered items:
1. A composition comprising at least one of an Active Compound and Botanical Powder, and at least one Delivery System.
2. The composition of item 1 wherein the Botanical Powder comprises at least one member selected from the group consisting of alfalfa (genus Medicago), amaranth (genus Amaranthus), argan (genus Argania), arrowroot, barley (genus Hordeum), lupine (genus Lupinus), oat (genus Avena), rice (genus Oryza), soya (genus Glycine), pea (genus Pisum), powders of cinnamon extract, powders of Citrus medica limonum extract, powders of Cucumis, powders of Glycyrrhiza, wheat (genus Triticum) baobab (genus Adansonia), hop (genus Humulus), maize (genus Zea), chick pea (genus Cicer), apple (genus Pyrus), citrus (genus citrus), and coconut (genus Cocos).
3. The composition of item 2 wherein the Botanical Powder comprises alfalfa.
4. The composition of item 2 wherein the Botanical Powder comprises amaranth.
5. The composition of item 2 wherein the Botanical Powder comprises argan.
6. The composition of item 2 wherein the Botanical Powder comprises arrowroot.
7. The composition of item 2 wherein the Botanical Powder comprises barley.
8. The composition of item 2 wherein the Botanical Powder comprises lupine.
9. The composition of item 2 wherein the Botanical Powder comprises oat.
10. The composition of item 2 wherein the Botanical Powder comprises rice.
11. The composition of item 2 wherein the Botanical Powder comprises soya.
12. The composition of item 2 wherein the Botanical Powder comprises wheat.
13. The composition of item 2 wherein the Botanical Powder comprises materials derived from any one or more parts of, or whole plant of, any plant within the group.
14. The composition of item 2 wherein the material comprises at least one of hydrolyzed extracts, partially hydrolyzed extracts, a substance derived from the Botanical Powder and combinations thereof.
15. The composition of item 1 wherein the Delivery System comprises at least one member selected from the group consisting of Cerasome, Liposome I and Liposome II.
16. The composition of item 15 wherein the Delivery System comprises Liposome I.
17. The composition of item 16 wherein the Liposome I comprises Phospholipids of type PL2.
18. The composition of item 15 further comprising at least one solvent selected from the group consisting of ethanol, 1,2-propanediol, 1,3-propanediol, 1,3-butylene glycol, and glycerol.
19. The composition of item 15 wherein the Delivery System comprises Liposome II and at least one solvent selected from the group consisting of 1,3 propanediol and glycerol.
20. The composition of item 15 wherein the composition is essentially free of Liposome when the Botanical Powder comprises at least one of Terminaliae fructus extract, Glycyrrhiza, Rosmarinus officinalis, Centella, Echinacea and Alpinia.
21. The composition of item 1 wherein the composition comprises at least one Active Compound and the Delivery System comprises Liposome II.
22. A Personal Care product comprising the composition of item 1.
23. A Cosmetic product comprising the composition of item 1.
24. The composition of item 1 wherein the composition has reduced sedimentation.
25. The composition of item 15 wherein the composition has reduced sedimentation.
26. Use of a Delivery System to reduce sedimentation in a Botanical Powder-Based Delivery System.
27. Use of a Delivery System of item 19 to deliver at least one Active Compound to the epidermis.

## Claims

**1.** A composition comprising at least one of an Active Compound and Botanical Powder, and at least one Delivery System,
wherein the Botanical Powder comprises at least one member selected from the group consisting of apple (genus Pyrus), rice (genus Oryza), soya (genus Glycine), coconut (genus Cocos) and citrus (genus citrus), and
wherein the Delivery System comprises at least one member selected from the group consisting of Cerasome, Liposome I and Liposome II.

**2.** The composition of Claim 1 wherein the Botanical Powder comprises materials derived from any one or more parts of, or whole plant of, any plant within the group.

**3.** The composition of Claim 2 wherein the material comprises at least one of hydrolyzed extracts, partially hydrolyzed extracts, a substance derived from the Botanical Powder and combinations thereof.

**4.** The composition of Claim 3 wherein the Delivery System comprises Liposome I.

**5.** The composition of Claim 4 wherein the Liposome I comprises Phospholipids of type PL2.

**7.** The composition of Claim 4 further comprising at least one solvent selected from the group consisting of ethanol, 1,2-propanediol, 1,3-propanediol, 1,3-butylene glycol, and glycerol.

**8.** The composition of Claim 4 wherein the Delivery System comprises Liposome II and at least one solvent selected from the group consisting of 1,3 propanediol and glycerol.

**9.** The composition of Claim 1 wherein the composition comprises at least one Active Compound and the Delivery System comprises Liposome II.

**10.** A Personal Care product or cosmetic product comprising the composition of Claim 1.

**11.** The composition of claim 1 or claim 4 wherein the composition has reduced sedimentation.

**12.** Use of a Delivery System to reduce sedimentation in a Botanical Powder-Based Delivery System.

**13.** Use of a Delivery System of Claim 8 to deliver at least one Active Compound to the epidermis.
